(19) Europäisches Patentamt
European Patent Office
Office européen des brevets

(11) EP 1 937 732 B1

(12) EUROPÄISCHE PATENTSCHRIFT

(45) Veröffentlichungstag und Bekanntmachung des Hinweises auf die Patenterteilung:
**18.03.2009 Patentblatt 2009/12**

(21) Anmeldenummer: **06793899.3**

(22) Anmeldetag: **29.09.2006**

(51) Int Cl.:
***C08F 220/18*** (2006.01) ***C08F 220/06*** (2006.01)
***A61K 8/81*** (2006.01)

(86) Internationale Anmeldenummer:
**PCT/EP2006/066871**

(87) Internationale Veröffentlichungsnummer:
**WO 2007/042405 (19.04.2007 Gazette 2007/16)**

(54) **HAARFESTIGER AUF BASIS VON T-BUTYLACRYLAT UND HYDROXYALKYLMETHACRYLAT**

HAIR FIXATIVE BASED ON T-BUTYL ACRYLATE AND HYDROXYALKYL METHACRYLATE

AGENT DE RENFORCEMENT CAPILLAIRE A BASE DE T-BUTYLACRYLATE ET D'HYDROXYALKYLMETHACRYLATE

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(30) Priorität: **07.10.2005 EP 05109363**

(43) Veröffentlichungstag der Anmeldung:
**02.07.2008 Patentblatt 2008/27**

(73) Patentinhaber: **BASF SE**
**67056 Ludwigshafen (DE)**

(72) Erfinder:
- **WINTER, Gabi**
  **Shanghai 201204 (CN)**
- **LAUBENDER, Matthias**
  **67105 Schifferstadt (DE)**

(56) Entgegenhaltungen:
**EP-A2- 0 379 082** **EP-A2- 0 815 839**
**WO-A-02/38638**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind Copolymere, die zwischen 25 und 80 Gew.-% tert.-Butyl(meth) acrylat, 2 bis 60 Gew.-% Hydroxyalkyl(meth)acrylat, 10 bis 40 Gew.-% einer anionischen oder anionogenen, radikalisch polymerisierbaren, olefinisch ungesättigten Verbindung, die Methacrylsäure ist oder umfasst,sowie gegebenenfalls bis zu 30 Gew.-% einer weiteren radikalisch polymerisierbaren, olefinisch ungesättigten Verbindung einpolymerisiert enthält. Weiterhin betrifft die vorliegende Erfindung die Verwendung solcher Copolymere in kosmetischen Zubereitungen sowie solche kosmetischen Zubereitungen an sich.

## Stand der Technik

Strengere Umweltauflagen und wachsendes ökologisches Bewusstsein fordern zunehmend immer geringere Anteile an flüchtigen organischen Komponenten (englisch: volatile organic compounds, VOC) in kosmetischen Aerosol-Zubereitungen wie beispielsweise Aerosol-Haarsprays.

Der VOC-Gehalt in Haarsprays wird im wesentlichen durch die nicht-wässrigen Lösungsmittel und die Treibmittel bestimmt. Daher wird gegenwärtig verstärkt anstelle von nicht-wässrigen Lösungsmittel auf Wasser als Lösungsmittel zurückgegriffen. Dieser Ersatz der organischen Lösungsmitteln bringt allerdings einige Probleme mit sich.

So sind Formulierungen der aus dem Stand der Technik bekannten filmbildenden Polymere, die entsprechende VOC-Auflagen erfüllen, beispielsweise nicht oder erst nach weiterer Verdünnung sprühbar und somit nur bedingt für die Verwendung in Haarsprays geeignet. Polymerfilme, die aus solchen Zubereitungen entstehen, weisen mitunter nicht die notwendige mechanische Qualität und somit ungenügende Festigungswirkung und schlechten Halt für das Haar auf.

DE 1928368 beschreibt Bindemittel für kosmetische Präparate, welche dadurch gekennzeichnet sind, dass es ein hydrophiles Polyacrylat und/oder -methacrylat enthält. Vorzugsweise ist das polymere Bindemittel ein Hydroxyalkyl- oder Hydroxyalkoxyalkylacrylat, beziehungsweise -methacrylat mit niederen Alkylresten. und gegebenenfalls niederen Alkoxyresten, Acryl- oder Methacrylamid, Diacetonacrylamid, Methylolacryl-oder Methylolmethacrylamid. Copolymere, die tert.-Butyl(meth)acrylate einpolymerisiert enthalten, werden nicht beschrieben.

EP-A 379 082 beschreibt Haarfestigungsmittel, welche als Filmbildner Copolymerisate auf der Basis von tert.-Butylacrylat und/oder tert.-Butylmethacrylat mit einem K-Wert von 10 bis 50 enthalten, die durch radikalische Polymerisation von 75 bis 99 Gew.% tert.-Butylacrylat und/oder tert.-Butylmethacrylat als Monomer A, 1 bis 25 Gew.% Acrylsäure und/oder Methacrylsäure als Monomer B und 0 bis 10 Gew.% eines weiteren radikalisch copolymerisierbaren Monomeren C erhältlich sind, wobei die Carboxylgruppen der Copolymerisate teilweise oder vollständig durch Amine neutralisiert sind. Copolymere, die Hydroxyalkyl(meth)acrylate einpolymerisiert enthalten, werden nicht beschrieben.

EP-A 638 306 beschreibt haarkosmetische VOC-80-Zubereitungen, die unter anderem Acrylpolymere oder Vinylesterpolymere enthalten. Copolymere, die tert.-Butyl(meth)acrylate einpolymerisiert enthalten, sind nicht beschrieben.

EP-A 705 595 beschreibt ein Acryl-Haarfixierungsharz welches, basierend auf dem Gesamtgewicht an Monomer, von 5 bis 95 Gew.-% wenigstens eines C1 bis C8-Alkyl(meth)acrylatmonomers, von 2 bis 70 Gew.-% wenigstens eines Hydroxyalkyl(meth)acrylatmonomers und von 2 bis 50 Gew.-% wenigestens eines C1 bis C8 monoethylenisch ungesättigten Monocarbonsäuremonomers umfasst. Copolymere, die tert.-Butyl(meth)acrylate einpolymerisiert enthalten, sind nicht beschrieben.

EP-A 605 686 beschreibt wäßrige Emulsionspolymere umfassend polymerisierte Reste von (a) einem oder mehreren ethylenisch ungesättigten sauren Monomeren, ausgewählt aus der Gruppe, die aus C3-C12-Mono- und -Dicarbonsäuren und den C1 -C8-Alkylhalbestern von Malein- und Fumarsäure und Kombinationen derselben besteht, vorliegend in einer Menge von 5 bis 35 Gew.-% des Polymers, und (b) einem oder mehreren wasserunlöslichen Comonomeren, ausgewählt aus der Gruppe, die aus C3-C12-Acrylaten und -Methacrylaten, C1 -C8-Alkyl-substituierten Acrylamiden und Methacrylamiden, Vinylestern von C3-C12-Carbonsäuren, Styrol und Kombinationen derselben besteht, vorliegend in einer Menge von 65 bis 95 Gew.-% des Polymers, und (c) fakultativ einem oder mehreren nichtionischen wasserlöslichen Comonomeren, ausgewählt aus einem oder mehreren der Gruppe, die aus wasserlöslichen Hydroxyalkylestern der Acryl- und Methacrylsäure, C1 -C4-Alkyl-C2-C4-aminoalkylestern der Acryl-und Methacrylsäure, Acrylamid und Methacrylamid, Dimethylacrylamid und - methacrylamid, N-Vinylpyrrolidon und Vinyl-Caprolactam besteht, vorliegend in einer Menge von bis zu 20 Gew.-% des Polymers. Copolymere, die tert.-Butyl(meth)acrylate einpolymerisiert enthalten, sind nicht beschrieben.

EP-A 985 401 beschreibt Haarfestigerpolymere aus (i) 5 bis 95 Gew.-% C1-C12-Alkyl (meth)acrylat, (ii) 2 bis 70 Gew.-% Hydroxyalkyl(meth)acrylat und (iii) 2 bis 50 Gew.-% C3-C8 monoethylenisch ungesättigter Monocarbonsäure. Copolymere, die tert.-Butyl(meth)acrylate einpolymerisiert enthalten, sind nicht beschrieben.

EP-A 985 405 und US 6,214,328 beschreiben Haarfestigerpoylmere aus (i) 5 bis 95 Gew.-% C1-C10-Alkyl (meth)acrylat, (ii) 0 bis 70 Gew.-% Hydroxyalkyl(meth)acrylat und (iii) 0 bis 50 Gew.-% C3-C8 monoethylenisch ungesättigter Monocarbonsäure und (iv) 1-25 Gew.-% monoethylenisch ungesättigter C4-C8-Dicarbonsäure. Copolymere,

die tert.-Butyl(meth)acrylate einpolymerisiert enthalten, sind nicht beschrieben.

EP-A 1 321 130 beschreibt haarkosmetische Zubereitungen die wenigstens ein (Meth)acrylat-Copolymer umfassen, welches (a) Butyl(meth)acrylat, (b) Hydroxyalkyl(meth)acrylat, (c) gegebenenfalls weitere Monomere einpolymerisiert enthält. Copolymere, die zugleich tert.-Butyl(meth)acrylat und Methacrylsäure einpolymerisiert enthalten, sind nicht beschrieben.

US 3,577,518 beschreibt alkoholische kosmetische Zubereitungen, die Copolymere aus Hydroxyalkyl(meth) acrylaten, Alkyl(meth)acrylaten und gegebenenfalls kationischen Monomeren enthalten. Copolymere, die tert.-Butyl (meth)acrylate einpolymerisiert enthalten, sind nicht beschrieben.

US 4,196,190 beschreibt Haarfestigerzubereitungen, die Copolymere aus 10-30 Gew.-% Alkylacrylaten, 41-60 Gew.-% Methylmethacrylat, 5.20 Gew.-% Hydroxyethylmethacrylat und 12-30 Gew.-% Methacrylsäure. Copolymere, die tert.-Butyl(meth)acrylate einpolymerisiert enthalten, sind nicht beschrieben.

US 5,589,157 beschreibt wässrige Zusammensetzungen, die Copolymere aus C1-C5-Alkylacrylat, C1-C5-Alkylmethacrylat und C3-C5 ethylenisch ungesättigter Carbonsäure enthält. Copolymere, die tert.-Butyl(meth)acrylate einpolymerisiert enthalten, sind nicht beschrieben.

WO 02/38638 beschreibt Acrylatpolymerisate mit einem K-Wert von 10-60 erhältlich durch radikalische Polymerisation von 30-99 Gew% tert-Butylacrylat und tert-Butylmethacrylat als Monomer A, 1 - 28 Gew.% Acrylsäure und/ oder Methacrylsäure als Monomer B und 0 - 60 Gew. % eines radikalisch copolymerisierbaren Monomeren oder einer radikalisch copolymerisierbaren Monomerenmischung als Monomer C, wobei mindestens eines der Monomeren C ein Homopolymerisat mit einer Glastemperatur kleiner als 30°C liefert. Copolymere, die Hydroxyalkyl(meth)acrylate einpolymerisiert enthalten, sind nicht beschrieben.

**Aufgabe und Lösung**

Eine Aufgabe der vorliegenden Erfindung bestand darin, Polymere für kosmetische, insbesondere haarkosmetische Zubereitungen bereitzustellen, welche als Pump- oder Aerosolspray in Lösungsmitteln oder Lösungsmittelgemischen mit erhöhtem Wasseranteil als klare Flüssigkeit gut formulierbar sind, deren Formulierungen in Form von kleinen gleichmäßigen Tröpfchen gut sprühbar sind und während und nach dem Aufbringen möglichst wenig zur Schaumbildung neigen und deren dann gebildete Filme nicht klebrig sind und gute mechanische Eigenschaften aufweisen.

Neben der guten Verträglichkeit mit den üblichen kosmetischen Inhaltsstoffen sollen die auf das Haar aufgetragenen Polymere schnell trocknen und dem Haar gute Festigung und längeren Halt auch bei erhöhter Luftfeuchtigkeit verleihen, gute Auswaschbarkeit aufweisen und sich als optisch klare VOC-55-Aerosole (d.h. mit einem VOC-Anteil von höchstens 55 Gew.-%) formulieren lassen. Weiterhin soll das behandelte Haar gute haptische Eigenschaften wie beispielsweise ein gutes Anfaßgefühl aufweisen.

Diese Aufgaben wurden überraschenderweise gelöst durch Copolymere, welches

a) zwischen 25 und 80 Gew.-% tert.-Butyl(meth)acrylat,

b) 2 bis 60 Gew.-% Hydroxyalkyl(meth)acrylat,

c) 10 bis 40 Gew.-% einer anionischen oder anionogenen, radikalisch polymerisierbaren Verbindung, die Methacrylsäure ist oder umfasst und

d) 0 bis 30 Gew.-% wenigstens einer weiteren radikalisch polymerisierbaren Verbindung

einpolymerisiert enthält, wobei sich die Mengen der Komponenten a) bis d) zu 100 Gew.-% addieren.

Im Rahmen der vorliegenden Erfindung umfasst der Ausdruck Alkyl geradkettige und verzweigte Alkylgruppen. Geeignete kurzkettige Alkylgruppen sind z.B. geradkettige oder verzweigte $C_1$-$C_{12}$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc..

Geeignete längerkettige $C_8$-$C_{30}$-Alkyl- bzw. $C_8$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z.B. n-Hexyl(en), n-Heptyl(en), n-Octyl(en), n-Nonyl (en), n-Decyl(en), n-Undecyl(en), n-Dodecyl(en), n-Tridecyl(en), n-Tetradecyl(en), n-Pentadecyl(en), n-Hexadecyl(en),

n-Heptadecyl(en), n-Octadecyl(en), n-Nonadecyl(en) etc..

Cycloalkyl steht vorzugsweise für $C_5$-$C_8$-Cycloalkyl, wie Cyclopentyl, Cyclohexyl, Cycloheptyl oder Cyclooctyl.

Der Ausdruck Heterocycloalkyl im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, COOR, $COO^-M^+$ und $NE^1E^2$, bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, - $SO_3H$, Sulfonat, $NE^1E^2$, Alkylen-$NE^1E^2$, Nitro, Cyano oder Halogen auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Arylalkyl steht für Gruppen, die sowohl Alkyl- als auch Arylreste enthalten, wobei diese Arylalkyl-Gruppen entweder über den Aryl- oder über den Alkylrest mit der sie tragenden Verbindung verknüpft sind.

Komponente a)

Bevorzugt enthält das erfindungsgemäße Copolymer, bezogen auf die Gesamtmenge der Komponenten a) bis d), mehr als 30, besonders bevorzugt wenigstens 40, ganz besonders bevorzugt wenigstens 45 und insbesondere wenigstens 50 und höchstens 75, besonders bevorzugt höchstens 70, ganz besonders bevorzugt höchstens 65 und insbesondere höchstens 60 Gew.-% tert.-Butyl(meth)acrylat a) einpolymerisiert.

Tert.-Butyl(meth)acrylat bedeutet tert.-Butylmethacrylat und/oder tert.-Butylacrylat.

Bevorzugt ist Komponente a) tert.-Butylacrylat oder eine Mischung aus tert.-Butylmethacrylat und tert.-Butylacrylat. Wird als Komponente eine Mischung aus tert.-Butylmethacrylat und tert.-Butylacrylat verwendet, so beträgt das Gewichtsverhältnis tert.-Butylmethacrylat zu tert.-Butylacrylat bevorzugt wenigstens 1:1,5, besonders bevorzugt wenigstens 1:2 und insbesondere wenigstens 1:2,5.

Komponente b)

Das erfindungsgemäße Copolymer enthält, bezogen auf die Gesamtmenge der Komponenten a) bis d), wenigstens 2, bevorzugt wenigstens 5, besonders bevorzugt wenigstens 10 und höchstens 60, bevorzugt höchstens 40 und besonders bevorzugt höchstens 35 Gew.-% Hydroxyalkyl(meth)acrylat b) einpolymerisiert. Hydroxyalkyl(meth)acrylate sind Ester der (Meth)acrylsäure mit Alkanen, die wenigstens 2 Hydroxygruppen tragen, insbesondere mit Alkandiolen.

Geeignete Hydroxyalkyl(meth)acrylate b) sind beispielsweise 2-Hydroxyethylacrylät, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat, Neopentylglykolmono(meth)acrylat, 1,5-Pentandiolmono(meth)acrylat, 1,6-Hexandiolmono(meth)acrylat und Mischungen davon.

Besonders bevorzugt ist Komponente b) ausgewählt aus der Gruppe bestehend aus Hydroxyethyl(meth)acrylat, 2-Hydroxypropyl(meth)acrylat, 3-Hydroxypropyl(meth)acrylat.

Insbesondere ist Komponente b) ausgewählt aus der Gruppe bestehend aus Hydroxyethylmethacrylat, 2-Hydroxypropylmethacrylat, 3-Hydroxypropylmethacrylat und Mischungen davon.

Ganz besonders bevorzugt als Komponente b) ist Hydroxyethylmethacrylat.

Komponente c)

Die erfindungsgemäßen Copolymere enthalten, bezogen auf das Gesamtgewicht der Komponenten a) bis d), 10 bis 40 Gew.-% einer anionischen oder anionogenen, radikalisch polymerisierbaren Verbindung c), die Methacrylsäure ist oder umfasst, einpolymerisiert.

Eine anionogene Verbindung ist eine Verbindung, die durch Deprotonierung mit Basen in die entsprechende anionische Form überführt werden kann.

Komponente c) kann außer Methacrylsäure weitere anionische oder anionogene, radikalisch polymerisierbare Verbindungen enthalten. Bevorzugt sind diese ausgewählt aus der Gruppe bestehend aus Acrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure, Fumarsäure und Mischungen davon. Komponente c) kann außer Methacrylsäure auch Halbester von monoethylenisch ungesättigten Dicarbonsäuren mit 4 bis 10 vorzugsweise 4 bis 6 C-Atomen, z. B. von Maleinsäure wie Maleinsäuremonomethylester enthalten.

Wenn Komponente c) außer Methacrylsäure weitere anionische oder anionogene, radikalisch polymerisierbare Verbindungen enthält, so sind diese bevorzugt ausgewählt aus der Gruppe bestehend aus Acrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und deren Mischungen, besonders bevorzugt Acrylsäure.

Enthält Komponente c) außer Methacrylsäure noch weitere anionische oder anionogene, radikalisch polymerisierbare Verbindungen, so beträgt das Gewichtsverhältnis von Methacrylsäure zu diesen Verbindungen, wie beispielsweise Acrylsäure, bevorzugt wenigstens 2:1, besonders bevorzugt wenigstens 2,5:1 und insbesondere wenigstens 3:1.

Komponenten c) sind auch die Salze der zuvor genannten Säuren, insbesondere die Natrium-, Kalium- und Ammoniumsalze. Die angegebenen Gewichtsanteile beziehen sich auf die Säureform.

Ein bevorzugter Gegenstand der Erfindung ist ein erfindungsgemäßes Copolymer wobei Komponente c) Methacrylsäure oder eine Mischung von Methacrylsäure und wenigstens einer weiteren monoethylenisch ungesättigten Carbonsäure ist. Insbesondere ist Komponente c) Methacrylsäure oder eine Mischung von Methacrylsäure und Acrylsäure.

Zur Herstellung der Copolymere kann Komponente c) in teilweise oder vollständig deprotonierter Form eingesetzt werden. Dann leiten sich deren Gegenionen vorzugsweise von Basen ab, wie sie im Folgenden zur Einstellung des pH-Werts bei der Polymerisation oder der erhaltenen Polymerisate beschrieben werden. Bevorzugt sind kosmetisch akzeptable Salze wie beispielsweise Alkali- (z.B. Natrium-, Kalium-) oder Ammoniumsalze.

Bevorzugt enthält das erfindungsgemäße Copolymer, bezogen auf die Gesamtmenge der Komponenten a) bis d), wenigstens 12, besonders bevorzugt wenigstens 15 und höchstens 35, besonders bevorzugt höchstens 30 Gew.-% der Komponente c) einpolymerisiert.

Komponente d)

Gegebenenfalls enthält das erfindungsgemäße Copolymer, bezogen auf die Gesamtmenge der Komponenten a) bis d), 0 bis 30 Gew.-% wenigstens einer weiteren radikalisch polymerisierbaren Verbindung d) einpolymerisiert.

Bevorzugt beträgt die Menge dieser einpolymerisierten Komponente d), bezogen auf die Gesamtmenge der Komponenten a) bis d), 0-20, besonders bevorzugt 0-10 und ganz besonders bevorzugt 0,5-5 Gew.-%.

Als Komponente d) kommen alle von den Komponenten a) bis c) verschiedenen, radikalisch polymerisierbaren Verbindungen in Betracht, die mit den Komponenten a) bis c) copolymerisiert werden können.

Bevorzugte von den Komponenten a), b) und c) verschiedene Komponenten d) sind im Folgenden angegeben.

d1) im Wesentlichen hydrophobe, nichtionische Verbindungen, bevorzugt Ester von Vinylalkohol oder Allylalkohol mit C1-C30-Monocarbonsäuren, Vinylether, Vinylaromaten, Vinylhalogenide, Vinylidenhalogenide, C2-C8-Monoolefine, nichtaromatische Kohlenwasserstoffe mit mindestens 2 konjugierten Doppelbindungen und Mischungen davon.

Geeignete Verbindungen d1) sind demnach beispielsweise Vinylformiat, Vinylacetat, Vinylpropionat, Vinyl-n-butyrat, Vinylstearat, Vinyllaurat, Styrol, α-Methylstyrol, o-Chlorstyrol, Acrylnitril, Methacrylnitril, Vinyltoluole, Vinylchlorid, Vinylidenchlorid, Vinylfluorid, Vinylidenfluorid, Ethylen, Propylen, Isobuten, Butadien, Isopren, Chloropren, Methyl-, Ethyl-, Butyl-, Dodecylvinylether und Mischungen davon.

Bevorzugte Verbindungen d1) sind auch die von a) verschiedenen Ester α,β-ethylenisch ungesättigter Mono- und Dicarbonsäuren mit C1-C30-Alkanolen, bevorzugt C1-C22-Alkanolen. Bevorzugt sind auch die Amide α,β-ethylenisch ungesättigter Mono- und Dicarbonsäuren mit Mono- und Dialkylaminen, die 1 bis 30 Kohlenstoffatome, bevorzugt 1 bis 22 Kohlenstoffatome pro Alkylrest aufweisen.

Vorzugsweise handelt es sich um Verbindungen der allgemeinen Formel III

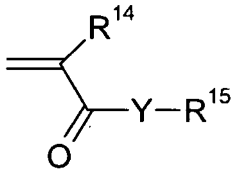

(III)

worin

$R^{14}$ für Wasserstoff oder C1- bis C8-Alkyl steht,
$R^{15}$ für einen geradkettigen oder verzweigten C1- bis C30-Alkylrest steht, und
Y für O oder $NR^{16}$ steht, wobei $R^{16}$ für Wasserstoff, C1- bis C8-Alkyl oder C5- bis C8-Cycloalkyl steht.

Vorzugsweise steht in der Formel III $R^{14}$ für Wasserstoff, Methyl oder Ethyl. Bevorzugt steht Y für O oder NH.

Geeignete Reste $R^{15}$ sind die zuvor genannten C1-C30-Alkylreste. Insbesondere steht $R^{15}$ für Methyl, Ethyl, n-Propyl, Isopropyl, n-Butyl, 2-Butyl, sec.-Butyl, tert.-Butyl, n-Pentyl, n-Hexyl, n-Heptyl, n-Octyl, Ethylhexyl, 1,1,3,3-Tetramethylbutyl, Undecyl, Lauryl, Tridecyl, Myristyl, Pentadecyl, Palmityl, Margarinyl, Stearyl, Palmitoleinyl, Oleyl oder Linolyl.

Insbesondere ist Komponente d1) ausgewählt unter Methyl(meth)acrylat, Methylethacrylat, Ethyl(meth)acrylat, n-Propyl(meth)acrylat, i-Propyl(meth)acrylat, nButyl(meth)acrylat, i-Butyl(meth)acrylat, sec- Butyl(meth)acrylat, 2-Pentyl (meth)acrylat, 3-Pentyl(meth)acrylat, Isopentyl(meth)acrylat, Neopentyl(meth)acrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth) acrylat, Tridecyl(meth)acrylat, Myristyl(meth)acrylat, Pentadecyl(meth)acrylat, Palmityl(meth)acrylat, Heptadecyl(meth) acrylat, Nonadecyl(meth)acrylat, Arrachinyl(meth)acrylat, Behenyl(meth)acrylat, Lignocerenyl(meth)acrylat, Cerotinyl (meth)acrylat, Melissinyl(meth)acrylat, Palmitoleinyl(meth)acrylat, Oleyl(meth)acrylat, Linolyl(meth)acrylat, Linolenyl (meth)acrylat, Stearyl(meth)acrylat, Lauryl(meth)acrylat, Phenoxyethyl(meth)acrylat, 4-t-Butylcyclohexylacrylat, Cyclohexyl(meth)acrylat, Ureido(meth)acrylat, Tetrahydrofurfuryl(meth)acrylat und deren Mischungen.

Komponente d1) kann auch ausgewählt werden unter (Meth)acrylamid, N-Methyl(meth)acrylamid, N-Ethyl (meth)acrylamid, N-n-Propyl-(meth)acrylamid, N-i-Propyl-(meth)acrylamid, N-(n-Butyl)methacrylamid, N-(sek.-Butyl) methacrylamid, N-(tert.-Butyl)methacrylamid, N-(n-Pentyl)(meth)acrylamid, N-(n-Hexyl)(meth)acrylamid, N-(n-Heptyl) (meth)acrylamid,tert.-Butyl(meth)acrylamid, n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth) acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth) acrylamid, Linolenyl(meth)acrylamid, Stearyl(meth)acrylamid, Lauryl(meth)acrylamid und Mischungen davon.

d2) Verbindungen mit mindestens einer ionogenen bzw. ionischen Gruppe pro Molekül Die Verbindungen d2) weisen mindestens eine ionogene bzw. ionische Gruppe pro Molekül auf, die bevorzugt ausgewählt ist unter Carboxylatgruppen und/oder Sulfonatgruppen und deren durch teilweise oder vollständige Neutralisation mit einer Base erhältlichen Salze, sowie tertiäre Aminogruppen, die teilweise oder vollständig protoniert und quaternisiert sein können. Geeignete Basen für die Neutralisation bzw. Säuren für die Protonierung und Alkylierungsmittel für die Quaternisierung sind dem Fachmann bekannt.

Geeignete Verbindungen d2) sind Acrylamidoalkansulfonsäuren und deren Salze, wie 2-Acrylamido-2-methylpropansulfonsäure und deren Alkalimetallsalze, z. B. deren Natrium- und Kaliumsalze.

Weitere geeignete Verbindungen d2) sind die Ester der zuvor genannten, $\alpha,\beta$-ethylenisch ungesättigten Mono- und Dicarbonsäuren mit C2- bis C12-Aminoalkoholen, welche am Aminstickstoff C1- bis C8-dialkyliert sind. Dazu zählen z. B. N,N-Dimethylaminomethyl-(meth)acrylat, N,N-Dimethylaminoethyl(meth)acrylat, N,N-Diethylaminoethyl(meth) acrylat, N,N-Dimethylaminopropyl(meth)acrylat, N,N-Diethylaminopropyl(meth)acrylat, N,N-Dimethylaminocyclohexyl (meth)acrylat etc. Bevorzugt werden N,N-Dimethylaminopropylacrylat und N,N-Dimethylaminopropyl(meth)acrylat eingesetzt.

Geeignete Verbindungen d2) sind weiterhin die Amide der zuvor genannten $\alpha,\beta$-ethylenisch ungesättigten Mono- und Dicarbonsäuren mit Diaminen, die eine tertiäre und ein primäre oder sekundäre Aminogruppe aufweisen. Dazu zählen z. B. N-[2-(dimethylamino)ethyl]- acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)- butyl]methacrylamid, N-[2-(diethylamino)ethyl]acrylamid, N-[4-(dimethylamino)cyclohexyl]acrylamid oder N-[4-(dimethylamino)cyclohexyl]methacrylamid.

Geeignete Verbindungen d2) sind weiterhin vinyl- und allylsubstituierte heteroaromatische Verbindungen, wie

2- und 4-Vinylpyridin, Allylpyridin, und bevorzugt N-Vinylheteroaromaten, wie N-Vinylimidazol oder N-Vinyl-2-methylimidazol.

d3) im Wesentlichen hydrophile, nichtionische Verbindungen, bevorzugt N-Vinylamide, N-Vinyllactame, primäre Amide α,β-ethylenisch ungesättigter Monocarbonsäuren, vinyl- und allylsubstituierte heteroaromatische Verbindungen, von b) verschiedene Ester α,β-ethylenisch ungesättigter Mono- und Dicarbonsäuren mit C1-C30-Alkandiolen, Ester und Amide α,β-ethylenisch ungesättigter Mono- und Dicarbonsäuren mit C2-C30-Aminoalkoholen, die eine primäre oder sekundäre Aminogruppe aufweisen, Polyetheracrylate und deren Mischungen.

Geeignete N-Vinyllactame d3) sind beispielsweise solche, die einen oder mehrere C1-C6-Alkylsubstituenten, wie Methyl, Ethyl, n-Propyl, Isopropyl, n-Butyl, sec.-Butyl, tert.-Butyl etc. aufweisen. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam und N-Vinyl-7-ethyl-2-caprolactam.

Geeignete Verbindungen d3) sind weiterhin primäre Amide der zuvor genannten α,β-ethylenisch ungesättigten Monocarbonsäuren, wie Acrylamid, Methacrylamid und E-thacrylamid.

Geeignete Verbindungen d3) sind auch die Ester der zuvor genannten Säuren mit Triolen und Polyolen, wie z. B. Glycerin, Erythrit, Pentaerythrit oder Sorbit. Geeignete Verbindungen d3) sind weiterhin Polyetheracrylate

Geeignete Komponenten e5) und e6) sind auch N-Vinylimidazole der allgemeinen Formel VII, worin $R^1$ bis $R^3$ für Wasserstoff, $C_1$-$C_4$-Alkyl oder Phenyl steht

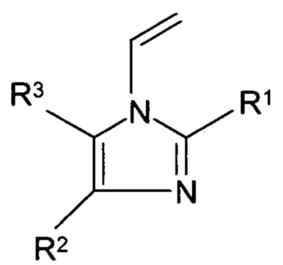

(VII)

Beispiele für Verbindungen der allgemeinen Formel VII sind folgender Tabelle 1 zu entnehmen:

Tabelle 1

| $R^1$ : | $R^2$ : | $R^3$ : |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |

(fortgesetzt)

| $R^1$ : | $R^2$ : | $R^3$ : |
|---|---|---|
| Me | H | Ph |
| Me = Methyl; Ph = Phenyl | | |

Die geladenen kationischen Gruppen lassen sich aus den Aminstickstoffen durch Quaternisierung mit sogenannten Alkylierungsmitteln erzeugen. Beispiele für geeignete Alkylierungsmittel sind $C_1$-$C_4$-Alkylhalogenide oder -sulfate, wie Ethylchlorid, Ethylbromid, Methylchlorid, Methylbromid, Dimethylsulfat und Diethylsulfat. Eine Quaternisierung kann im Allgemeinen sowohl vor als auch nach der Polymerisation erfolgen.

d4) Verbindungen mit wenigstens zwei radikalisch polymerisierbaren Doppelbindungen Als Komponente d) kommen auch Verbindungen mit wenigstens zwei radikalisch polymerisierbaren, olefinisch ungesättigten Doppelbindungen pro Molekül in Betracht. Solche Verbindungen werden üblicherweise als Vernetzer bezeichnet.

Radikalisch polymerisierbare, olefinisch ungesättigte Doppelbindungen sind beispielsweise Alkenylgruppen, die formal durch Ablösen eines H-Atoms aus einem Alken entstehen. Dazu gehören Vinyl- ($-CH=CH_2$), 1-Propenyl ($-CH=CH-CH_3$), 2-Propenyl oder Allyl ($-CH_2-CH=CH_2$), 1-Butenyl ($-CH=CH-CH_2-CH_3$) usw..

Auch Alkylidengruppen, also Gruppen, die mit einem Kohlenstoffatom eines Moleküls durch eine Doppelbindung verknüpft sind, gehören zu den radikalisch polymerisierbaren, olefinisch ungesättigte Doppelbindungen (Beispiel Ethyliden: $=CHCH_3$).

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei olefinisch ungesättigte Gruppen.

Beispiele für die zugrundeliegenden Alkohole sind zweiwertige Alkohole wie 1,2-Ethandiol, 1,2-Propandiol, 1,3-propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden.

Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten $C_3$- bis $C_6$-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Geeignet sind auch Alkylenbisacrylamide wie Methylenbisacrylamid und N,N'-(2,2-) butan und 1,1'-bis-(3,3'-vinylbenzimidazolith-2-on)-1,4-butan.

Andere geeignete Vernetzer sind beispielsweise Alkylenglykoldi(meth)acrylate wie Ethylenglykoldiacrylat, Ethylenglykoldimethacrylat, Tetraethlyenglykolacrylat, Tetraethylenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Vernetzer.

Weitere geeignete Vernetzer sind Divinyldioxan, Tetraallylsilan oder Tetravinylsilan.

Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Allylmethacrylat, Pentaerythrittriallylether, Methylenbisacrylamid, N,N'-Divinylethylenharnstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Selbstverständlich können auch Mischungen der vorgenannten Verbindungen eingesetzt werden. Dier Vernetzer ist vorzugsweise im Reaktionsmedium löslich. Ist die Löslichkeit des Vernetzers im Reaktionsmedium gering, so kann er in einem Monomeren oder in einer Monomerenmischung gelöst werden oder aber in einem Lösungsmittel gelöst zudosiert werden, das sich mit dem Reaktionsmedium mischt. Besonders bevorzugt sind solche Vernetzer, die in der Monomermischung löslich sind.

Herstellung der Copolymere

Die Herstellung der erfindungsgemäßen Copolymere erfolgt nach üblichen, dem Fachmann bekannten Verfahren, z.B. durch Lösungs-, Fällungs-, Suspensions- oder Emulsionspolymerisation.

Bevorzugt ist die Herstellung durch Lösungs- oder Emulsionspolymerisation.

Bevorzugte Lösemittel für die Lösungspolymerisation sind Alkohole oder Mischungen aus Alkoholen und Wasser., wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan.

Besonders bevorzugt ist die Polymerisation in Alkohol oder einem Wasser/AlkoholGemisch, beispielsweise in einem Wasser/Ethanol-Gemisch. Bei der Verwendung von Wasser als Lösungsmittel-Bestandteil wird bevorzugt entsalztes Wasser eingesetzt.

Besonders bevorzugt werden die erfindungsgemäßen Copolymere in bekannter Weise durch radikalisch initiierte wässrige Emulsionspolymerisation der Monomeren a), b) und gegebenenfalls c) hergestellt.

Emulsionspolymerisation

Die Methode der radikalisch initiierten wässrigen Emulsionspolymerisation ist vielfach vorbeschrieben und dem Fachmann daher hinreichend bekannt [vgl. z.B. Encyclopedia of Polymer Science and Engineering, Vol. 8, Seiten 659 bis 677, John Wiley & Sons, Inc., 1987; D.C. Blackley, Emulsion Polymerisation, Seiten 155 bis 465, Applied Science Publishers, Ltd., Essex, 1975; D.C. Blackley, Polymer Latices, 2nd Edition, Vol. 1, Seiten 33 bis 415, Chapman & Hall, 1997; H. Warson, The Applications of Synthetic Resin Emulsions, Seiten 49 bis 244, Ernest Benn, Ltd., London, 1972; D. Diederich, Chemie in unserer Zeit 1990, 24, Seiten 135 bis 142, Verlag Chemie, Weinheim; J. Piirma, Emulsion Polymerisation, Seiten 1 bis 287, Academic Press, 1982; F. Hölscher, Dispersionen synthetischer Hochpolymerer, Seiten 1 bis 160, Springer-Verlag, Berlin, 1969 und die DE-A 40 03 422].

Die radikalisch initiierte wässrige Emulsionspolymerisation erfolgt üblicherweise so, dass man die Monomeren, in der Regel in Gegenwart von Dispergiermitteln, in wässrigem Medium dispers verteilt und mittels wenigstens eines radikalischen Polymerisationsinitiators polymerisiert.

Initiatoren

Als radikalische Polymerisationsinitiatoren kommen alle diejenigen Initiatoren in Betracht, die in der Lage sind,

eine radikalische wässrige Emulsionspolymerisation auszulösen.

Es kann sich dabei prinzipiell sowohl um Peroxide als auch um Azoverbindungen handeln. Diese können anorganischer öder organischer Natur sein. Selbstverständlich kommen auch Redoxinitiatorsysteme in Betracht.

Als Peroxide können prinzipiell anorganische Peroxide, wie Wasserstoffperoxid oder Peroxodisulfate, wie die Mono- oder Di-Alkalimetall- oder Ammoniumsalze der Peroxodischwefelsäure, wie beispielsweise deren Mono- und Di-Natrium-, -Kalium- oder Ammoniumsalze oder organische Peroxide, wie Alkylhydroperoxide, beispielsweise tert.-Butyl-, p-Menthyl- oder Cumylhydroperoxid, tert.-Butylperpivalat sowie Dialkyl- oder Diarylperoxide, wie Di-tert.-Butyl- oder Di-Cumylperoxid, 2,5-Dimethyl-2,5-di(t)butylperoxy(hexan) oder Dibenzoylperoxid eingesetzt werden.

Als Azoverbindung finden im wesentlichen 2,2'-Azobis(isobutyronitril), 2,2'-Azobis(2,4-dimethylvaleronitril) und 2,2'-Azobis(amidinopropyl)dihydrochlorid (AIBA, entspricht V-50TM von Wako Chemicals), 1,1'-Azo-bis-(1-cyclohexancarbonitril), 2,2'-Azobis(2-amidinopropan)salze, 4,4'-Azobis(4-Cyanovaleriansäure) oder 2-(Carbamoylazo)-isobutyronitril Verwendung.

Als Oxidationsmittel für Redoxinitiatorsysteme kommen im wesentlichen die oben genannten Peroxide in Betracht. Als entsprechende Reduktionsmittel können Schwefelverbindungen mit niedriger Oxidationsstufe, wie Alkalisulfite, beispielsweise Kalium-und/oder Natriumsulfit, Alkalihydrogensulfite, beispielsweise Kalium- und/oder Natriumhydrogensulfit, Alkalimetabisulfite, beispielsweise Kalium- und/oder Natriummetabisulfit, Formaldehydsulfoxylate, beispielsweise Kalium- und/oder Natriumformaldehydsulfoxylat, Alkalisalze, speziell Kalium- und/oder Natriumsalze aliphatischer Sulfinsäuren und Alkalimetallhydrogensulfide, wie beispielsweise Kalium- und/oder Natriumhydrogensulfid, Salze mehrwertiger Metalle, wie Eisen-(II)-sulfat, Eisen-(II)-Ammoniumsulfat, Eisen-(II)-phosphat, Endiole, wie Dihydroxymaleinsäure, Benzoin und/oder Ascorbinsäure sowie reduzierende Saccharide, wie Sorbose, Glucose, Fructose und/ oder Dihydroxyaceton eingesetzt werden.

Die Initiatoren werden üblicherweise in Mengen von 0,001 bis 10, vorzugsweise 0,02 bis 5 Gew.-%, bezogen auf die zu polymerisierenden Monomeren, eingesetzt.

Tenside und Schutzkolloide

Die Emulsionspolymerisation wird üblicherweise in Gegenwart von Tensiden und/oder Schutzkolloiden durchgeführt.

Bei der Herstellung der erfindungsgemäßen Copolymeren wird wenigstens ein Dispergierhilfsmittel mitverwendet, das sowohl die Monomertröpfchen als auch Polymerisatteilchen in der wässrigen Phase dispers verteilt halten kann und so die Stabilität der erzeugten wässrigen Polymerisatdispersion gewährleistet. Als solche kommen sowohl die zur Durchführung von radikalischen wässrigen Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloiden findet sich in Houben-Weyl, Methoden der organischen Chemie, Band XIV/1, Makromolekulare Stoffe, Seiten 411 bis 420, Georg-Thieme-Verlag, Stuttgart, 1961.

Selbstverständlich können auch Gemische aus Emulgatoren und/oder Schutzkolloiden verwendet werden. Vorzugsweise werden als Dispergierhilfsmittel ausschließlich Emulgatoren eingesetzt, deren relative Molekulargewichte im Unterschied zu den Schutzkolloiden üblicherweise unter 1000 liegen. Sie können sowohl anionischer, kationischer oder nichtionischer Natur sein. Selbstverständlich müssen im Falle der Verwendung von Gemischen grenzflächenaktiver Substanzen die Einzelkomponenten miteinander verträglich sein, was im Zweifelsfall an Hand weniger Vorversuche überprüft werden kann. Im allgemeinen sind anionische Emulgatoren untereinander und mit nichtionischen Emulgatoren verträglich. Desgleichen gilt auch für kationische Emulgatoren, während anionische und kationische Emulgatoren meistens nicht miteinander verträglich sind.

Gebräuchliche Emulgatoren sind z.B. ethoxilierte Mono-, Di- und Tri-Alkylphenole (EO-Grad: 3 bis 50, Alkylrest: C4 bis C12), ethoxilierte Fettalkohole (EO-Grad: 3 bis 50; Alkyl-rest: C8 bis C36) sowie Alkalimetall- und Ammoniumsalze von Alkylsulfaten (Alkylrest: C8 bis C12), von Schwefelsäurehalbestern ethoxylierter Alkanole (EO-Grad: 4 bis 30, Alkylrest: C12 bis C18) und ethoxilierter Alkylphenole (EO-Grad: 3 bis 50, Alkylrest: C4 bis C12), von Alkylsulfonsäuren (Alkylrest: C12 bis C18) und von Alkylarylsulfonsäuren (Alkylrest: C9 bis C18). Weitere geeignete Emulgatoren finden sich in Houben-Weyl, Methoden der organischen Chemie, Band XIV/1, Makromolekulare Stoffe, Seiten 192 bis 208, Georg-Thieme-Verlag, Stuttgart, 1961.

Als grenzflächenaktive Substanzen haben sich ferner Verbindungen der allgemeinen Formel II

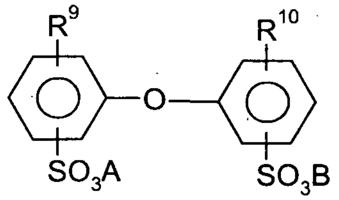

(II),

worin $R^9$ und $R^{10}$ C4- bis C24-Alkyl bedeuten und einer der Reste $R^9$ oder $R^{10}$ auch für Wasserstoff stehen kann, und A und B Alkalimetallionen und/oder Ammoniumionen sein können, erwiesen. In der allgemeinen Formel II bedeuten $R^9$ und $R^{10}$ bevorzugt lineare oder verzweigte Alkylreste mit 6 bis 18 C-Atomen, insbesondere mit 6, 12 und 16 C-Atomen oder H-Atome, wobei $R^9$ und $R^{10}$ nicht beide gleichzeitig H-Atome sind. A und B sind bevorzugt Natrium-, Kalium -oder Ammoniumionen, wobei Natriumionen besonders bevorzugt ist. Besonders vorteilhaft sind Verbindungen II, in denen A und B Natriumionen, $R^9$ ein verzweigter Alkylrest mit 12 C-Atomen und R10 ein H-Atom oder $R^9$ sind. Häufig werden technische Gemische verwendet, die einen Anteil von 50 bis 90 Gew.-% des monoalkylierten Produktes aufweisen, beispielsweise Dowfax®2A1 (Dow Chemical Company). Die Verbindungen II sind allgemein bekannt, z.B. aus US-A 4 269 749, und im Handel erhältlich.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate, Alkylglykolalkoxylate und diglykolalkoxylate und Alkylethercarboxylate können zwischen 1 bis 10 Ethylenoxid oder Propylenoxid-Einheiten, bevorzugt 1 bis 3 Ethylenoxideinheiten im Molekül aufweisen.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside, Alkylglykolalkoxylate und diglykolalkoxylate oder Sorbitanetherester geeignet.

Geeignete Tenside sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

In der Regel beträgt die Menge an eingesetztem Dispergierhilfsmittel 0,1 bis 5 Gew.-%, bevorzugt 1 bis 3 Gew.-%, jeweils bezogen auf die Gesamtmenge der radikalisch zu polymerisierenden Monomeren. Häufig ist es günstig, wenn eine Teil- oder die Gesamtmenge des Dispergierhilfsmittels dem fluiden Reaktionsmedium vor der Initiierung der radikalischen Polymerisation zugeführt wird. Darüber hinaus kann eine Teil- oder die Gesamtmenge des Dispergierhilfsmittels dem Reaktionsmedium vorteilhaft auch gemeinsam mit den zu polymerisierenden Monomeren, insbesondere in Form einer wässrigen Monomerenemulsion während der Polymerisation zugeführt werden.

Bevorzugt ist eine Kombination aus ionischen und nicht ionischen Dispergierhilfsmitteln.

Regler

Mit Hilfe von sogenannten Reglern kann das Molekulargewicht von Polymeren beeinflusst werden. Als Regler werden bevorzugt Alkanthiole eingesetzt. Es können auch Gemische von mehreren Reglern eingesetzt werden.

Als Alkanthiole werden lineare und verzweigte Alkanthiole mit einer C-Kettenlänge von C10 bis C22 eingesetzt. Besonders bevorzugt sind lineare Alkanthiole, weiterhin bevorzugt sind Alkanthiole mit einer Kettenlänge von C12 bis C22, insbesondere von C12 bis C18. Als bevorzugte Alkanthiole seien genannt n-Decanthiol, n-Dodecanthiol, tert.-Dodecanthiol, n-Tetradecanthiol, n-Pentadecanthiol, n-Hexadecanthiol, n-Heptadecanthiol, n-Octadecanthiol, n-Nonadecanthiol, n-Eicosanthiol, n-Docosanthiol. Besonders bevorzugt sind lineare, geradzahlige Alkanthiole. Die Alkanthiole können auch in Mischungen eingesetzt werden. Die Alkanthiole werden üblicherweise in Mengen von 0,1 bis 5 Gew.-%, insbesondere 0,25 bis 2 Gew.-% bezogen auf die zu polymerisierenden Monomere eingesetzt. Üblicherweise werden die Alkanthiole zusammen mit den Monomeren der Polymerisation zugesetzt.

Insbesondere bevorzugt als Regler ist n-Dodecanthiol (n-Dodecylmercaptan).

Wasserstoffperoxidbehandlung

Werden bei der Polymerisation Alkanthiole eingesetzt, ist gegebenenfalls eine anschließende Wasserstoffperoxidbehandlung erforderlich, um geruchlich neutrale Polymerisate zu erhalten. Für diese sich an die Polymerisation anschließende Wasserstoffperoxidbehandlung werden üblicherweise 0,01 bis 2,0 Gew.-%, insbesondere 0,02 bis 1,0 Gew.-%, bevorzugt 0,3 bis 0,8 Gew.-%, weiterhin bevorzugt 0,03 bis 0,15 Gew.-% Wasserstoffperoxid, bezogen auf die Menge der zu polymerisierenden Monomere eingesetzt. Es hat sich als vorteilhaft erwiesen, die Wasserstoffperoxidbehandlung bei einer Temperatur von 20 bis 100°C, insbesondere von 30 bis 80°C durchzuführen. Die Wasserstoffperoxidbehandlung wird üblicherweise für eine Dauer von 15min bis 240 min, insbesondere von 30 min bis 90 min durchgeführt.

Werden Alkanthiole mit einer C-Kettenlänge von C14 bis C22 eingesetzt, kann die Wasserstoffperoxidbehandlung entfallen. In einer weiteren Ausführungsform der Erfindung kann jedoch auch bei der Verwednung von Alkanthiolen mit einer Kettenlänge von C14 bis C22 eine Wasserstoffperoxidbehandlung angeschlossen werden.

Durchführung der Emulsionspolymerisation

Die Emulsionspolymerisation erfolgt üblicherweise unter Sauerstoffausschluß, beispielsweise unter Stickstoff- oder Argonatmosphäre, bei Temperaturen im Bereich von 20 bis 200 °C. Vorteilhaft sind Polymerisationstemperaturen im Bereich von 50 bis 130, insbesondere von 70 bis 95 °C.

Bei der radikalisch initiierten Emulsionspolymerisation ist insbesondere bei höheren Temperaturen zur Vermeidung von Koagulatbildung darauf zu achten, dass das Polymerisationsgemisch nicht siedet. Dies kann beispielsweise dadurch vermieden werden, dass die Polymerisationsreaktion bei einem Inertgasdruck erfolgt, welcher höher ist als der Dampfdruck des Polymerisationsgemisches, beispielsweise 1,2 bar, 1,5 bar, 2 bar, 3 bar, 5 bar, 10 bar oder noch höher (jeweils Absolutwerte). Die Polymerisation kann diskontinuierlich, semikontinuierlich oder kontinuierlich durchgeführt werden. Häufig erfolgt die Polymerisation bzw. die Monomeren- und die Reglerdosierung semikontinuierlich nach dem Zulaufverfahren.

Die Mengen an Monomeren und Dispergiermittel wählt man zweckmäßigerweise so, daß man eine 30 bis 80 gew.-%ige Dispersion der Copolymerisate enthält. Vorzugsweise dosiert man zumindest einen Teil der Monomere, Initiatoren und gegebenenfalls Regler während der Polymerisation gleichmäßig in das Reaktionsgefäß (Zulauffahrweise). Die Monomere und der Initiator können jedoch auch im Reaktor vorgelegt und polymerisiert werden, wobei gegebenenfalls gekühlt werden muß.

Gemäß einer bevorzugten Ausführungsform führt man die Polymerisation unter Verwendung eines Saatlatex durch. Zweckmäßigerweise wird der Saatlatex aus den zu polymerisierenden Polymeren in der ersten Polymerisationsphase in üblicher Weise hergestellt. Anschließend gibt man den verbleibenden Teil der Monomerenmischung zu, vorzugsweise nach dem Zulaufverfahren.

Die Polymerisationsreaktion erfolgt vorteilhaft bis zu einem Monomerenumsatz von wenigstens 95 Gew.-%, bevorzugt wenigstens 98 Gew.-%, besonders bevorzugt wenigstens 99 Gew.-%.

Häufig ist es sinnvoll, wenn die erhaltene wässrige Polymerisatdispersion zur weiteren Absenkung der nicht umgesetzten Monomerenmenge einem Nachpolymerisationsschritt unterzogen wird. Diese Maßnahme ist dem Fachmann bekannt (beispielsweise EP-B 3957, EP-B 28 348, EP-B 563 726, EP-A 764 699, EP-A 76 180, DE-A 3718 520, DE-A 3834734, DE-A 4232194, DE-A 19529599, DE-A 19741187, DE-A 19839199, DE-A 19840586, WO 95/33775 oder US 4529753).

Aufbereitung der Dispersionen

Wird das Copolymer durch Emulsionspolymerisation hergestellt, kann die erhaltene Dispersion entweder direkt in wässrige, wässrig-alkoholische oder alkoholische haarkosmetische Zubereitungen eingearbeitet werden oder es erfolgt eine Trocknung der Dispersion, z.B. Sprühtrocknung, Fluidized Spray Drying, Walzentrocknung oder Gefriertrocknung, so daß das Copolymer als Pulver verwendet und verarbeitet werden kann.

Bevorzugt wird die Sprühtrocknung als Trocknungsmethode eingesetzt. Die so erhaltenen Polymer-Trockenpulver lassen sich vorteilhafterweise durch Lösen bzw. Redispergieren in Wasser erneut in eine wässrige Lösung bzw. Dispersion überführen.

Pulverförmige Copolymere haben den Vorteil einer besseren Lagerfähigkeit, einer einfacheren Transportmöglichkeit und zeigen in der Regel eine geringere Neigung für Keimbefall.

Selbstverständlich ist es auch möglich, die wässrige Polymerisatdispersion vor oder nach dem Nachpolymerisationsschritt einer dem Fachmann ebenfalls bekannten Inertgas- und/oder Wasserdampfstrippung zu unterziehen. Bevorzugt erfolgt dieser Strippvorgang nach dem Nachpolymerisationsschritt. Wie in EP-A 805 169 beschrieben, ist eine Teilneutralisation der Dispersion auf einen pH-Wert im Bereich von 5 bis 7, bevorzugt auf einen pH-Wert im Bereich von 5,5 bis 6,5 vor der physikalischen Desodorierung von Vorteil.

Zur Teilneutralisation bis pH 5-7 könnnen auch alle im Folgenden unter "Neutralisation" genannten Basen verwendet werden.

Neutralisation

Für bestimmte Verwendungen ist es von Vorteil, wenn die Copolymeren wenigstens teilweise neutralisiert werden. Insbesondere zur Verwendung der Copolymeren in (Haar-)kosmetischen Zubereitungen ist eine teilweise oder vollständige Neutralisation der Copolymer-Dispersionen vorteilhaft.
Zur Neutralisation eignen sich Alkali- bzw. Ammoniumcarbonat oder - hydrogencarbonat.
Die Neutralisation kann auch erfolgen mit

- einem Mono-, Di- oder Trialkanolamin mit 2 bis 5 Kohlenstoffatomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,

- einem Alkandiolamin mit 2 bis 5 Kohlenstoffatomen, beispielsweise 2-Amino-2-methylpropan-1,3-diol und 2-Amino-2-ethylpropan-1,3-diol, oder

- einem primären, sekundären oder tertiären Alkylamin mit insgesamt 5 bis 10 Kohlenstoffatomen, beispielsweise N, N-Diethylpropylamin oder 3-Diethylamino-1-propylamin.

Als Alkalimetallhydroxide eignen sich zur Neutralisation vor allem Natrium-, oder Kalium- sowie Ammoniumhydroxid.
Häufig werden gute Neutralisationsergebnisse mit 2-Amino-2-methylpropanol, Triisopropanolamin, 2-Amino-2-ethylpropan-1,3-diol, N,N-Dimethylaminoethanol oder 3-Diethylamino-1-propylamin erhalten.
Zur Neutralisation der Polymere in den erfindungsgemäßen Zubereitungen und Mitteln sind inbesondere auch Aminogruppen enthaltende Silikonpolymere geeignet. Geeignete Aminogruppen enthaltende Silikonpolymere sind beispielsweise die Silikon-Aminopolyalkylenoxid-Blockcopolymere der WO 97/32917, die Produkte SilsoftÒA-843 (Dimethicone Bisamino Hydroxypropyl Copolyol) und SilsoftÒA-858 (Trimethylsilyl Amodimethicone Copolymer) (beide Fa. Witco). Weiterhin sind auch die Neutralisationspolymere der EP-A 1035144 und insbesondere die silikonhaltigen Neutralisationspolymere des Anspruchs 12 der EP-A 1035144 geeignet.
In einer bevorzugten Ausführungsform sind die Copolymere beispielsweise zu mindestens 10, bevorzugt zu mindestens 30, weiter bevorzugt zu mindestens 40, besonders bevorzugt zu mindestens 50, ganz besonders bevorzugt zu mindestens 70 und insbesondere zu mindestens 95 % neutralisiert.
In einer besonders bevorzugten Ausführungsform sind die Polymere zu mindestens 99% neutralisiert. Am meisten bevorzugt ist die Neutralisation zu mindestens 100%.
Es ist weiterhin möglich, wenn das Neutralisationsmittel in mehr als äquivalenter Menge zugesetzt wird, wobei unter äquivalenter Menge die Menge verstanden wird, die benötigt wird, um alle neutralisierbaren Gruppen des Copolymers zu neutralisieren.

Konservierung der Dispersionen

Zur Stabilisierung und Konservierung der Dispersionspolymere werden übliche Konservierungsmittel eingesetzt. Bevorzugt wird Wasserstoffperoxid eingesetzt.

K-Wert

Der K-Wert der erfindungsgemäßen Copolymere liegt im Bereich von 15 bis 120, bevorzugt von 25 bis 75 und besonders bevorzugt von 25 bis 55. Am meisten bevorzugt sind K-Werte im Bereich von 25 bis 45 (Bestimmung nach Fikentscher, Cellulosechemie, Bd. 13, S. 58 bis 64 (1932)).
Möglichkeiten, den K-Wert der Copolymere auf einen gewünschten Wert einzustellen, sind dem Fachmann bekannt. Beispielsweise sind dies die Polymerisationstemperatur, die Initiatormenge oder die Verwendung von Kettenübertragungsreagenzien (Reglern).

**Kosmetische Zubereitungen**

Der Begriff VOC ist dem Fachmann bekannt. VOC (engl.: volatile organic compounds - VOC) sind organisch chemische Verbindungen, welche bei Normaldruck in einem Bereich bis ca. 260°C sieden und somit gasförmig in die Raumluft gelangen können. Zu den flüchtigen organischen Verbindungen zählen zahlreiche Lösemittel und Treibmittel.

**Klassifizierung von organischen Verbindungen im Innenraum** (nach: WHO - World Health Organization 1989)

| **Verbindungen** | **Abkürzung** | **Siedepunktsbereich [°Celsius]** |
|---|---|---|
| leichtflüchtige organische Verbindungen (engl.: very volatile organic compounds) | VVOC | < 0 bis 50°C |
| flüchtige organische Verbindungen (engl.: volatile organic compounds) | VOC | 50 bis 260°C |
| Mittel- bis schwerflüchtige organische Verbindungen (engl.: semi volatile organic compounds) | SVOC | 260 bis 380°C |

Die erfindungsgemäßen Copolymere eignen sich hervorragend zur Herstellung kosmetischer, insbesondere haut- und/oder haarkosmetischer Zubereitungen. Sie dienen dabei z.B. als polymere Filmbildner. Sie sind universell in den verschiedensten kosmetischen, bevorzugt haarkosmetischen Zubereitungen einsetzbar und einformulierbar und mit den üblichen Komponenten verträglich.

Die erfindungsgemäßen Copolymere eignen sich vorteilhaft zur Erzeugung elastischer Frisuren bei gleichzeitig starker Festigung, auch bei hoher Luftfeuchtigkeit. Die erfindungsgemäßen Copolymere zeichnen sich durch gute Treibgasverträglichkeit, gute Löslichkeit in wässrig/alkoholischen Lösungsmittelgemischen, insbesondere durch die Eignung für einen Einsatz als optisch klare Low-VOC-Formulierungen und durch gute Auswaschbarkeit und Auskämmbarkeit ohne Flaking-Effekt aus. Zudem verbessern sie mit ihnen behandeltes Haar in seinen sensorisch erfassbaren Eigenschaften wie Griff, Volumen oder Handhabbarkeit. Haarsprayformulierungen auf Basis der erfindungsgemäßen Copolymere zeichnen sich durch gute Sprühbarkeit und gute rheologische Eigenschaften und äußerst geringe Klebrigkeit der resultierenden Filme aus. Die die erfindungsgemäßen Copolymere enthaltenden erfindungsgemäßen kosmetischen, bevorzugt haarkosmetischen Zubereitungen neigen nach dem Aufbringen nicht zur Schaumbildung. Neben der guten Verträglichkeit mit den üblichen kosmetischen Inhaltsstoffen trocknen die aufgetragenen erfindungsgemäßen Copolymere schnell.

Ein weiterer Gegenstand der vorliegenden Erfindung ist demnach die Verwendung der erfindungsgemäßen Copolymere in kosmetischen Zubereitungen sowie solche kosmetischen Zubereitungen an sich.

Kosmetisch akzeptabler Träger B

Bevorzugt handelt es sich bei den erfindungsgemäßen kosmetischen Zubereitungen um wässrige Zubereitungen, die wenigstens 10, bevorzugt wenigstens 20 und besonders bevorzugt wenigstens 30 Gew.-% Wasser enthalten. Bevorzugt enthalten die erfindungsgemäßen kosmetischen Zubereitungen höchstens 80, bevorzugt höchstens 55 Gew.-% flüchtige organische Bestandteile.

Eine besonders bevorzugte Ausführungsform sind demnach erfindungsgemäße kosmetische Zubereitungen, bei denen der Anteil an flüchtigen organischen Komponenten höchstens 55 Gew.-%, bezogen auf die kosmetische Zubereitung, beträgt.

Die erfindungsgemäßen kosmetischen Zubereitungen weisen neben Wasser und den erfindungsgemäßen Copolymeren weiterhin wenigstens einen kosmetisch akzeptablen Träger B auf, der ausgewählt ist unter

i) wassermischbaren organischen Lösungsmitteln, vorzugsweise $C_2$-$C_4$-Alkanolen, insbesondere Ethanol,
ii) Ölen, Fetten, Wachsen,
iii) von ii) verschiedenen Estern von $C_6$-$C_{30}$-Monocarbonsäuren mit ein-, zwei-oder dreiwertigen Alkoholen,
iv) gesättigten acyclischen und cyclischen Kohlenwasserstoffen,
v) Fettsäuren,
vi) Fettalkoholen,
vii) Treibmitteln (Treibgasen) und
viii) Mischungen davon.

Geeignete Träger B und weitere, vorteilhaft zu verwendende Wirk- und Zusatzstoffe sind im Folgenden detailliert beschrieben.

Geeignete kosmetisch und pharmazeutisch verträgliche Öl- bzw. Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Die erfindungsgemäßen Zubereitungen können z.B. als kosmetisch akzeptablen Träger B eine Öl- bzw. Fettkomponente aufweisen, die ausgewählt ist unter:Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc..

Bei den erfindungsgemäßen kosmetischen Zubereitungen kann es sich um hautkosmetische, haarkosmetische oder dermatologische, hygienische oder pharmazeutische Zubereitungen handeln. Aufgrund ihrer filmbildenden und flexiblen Eigenschaften eignen sich die zuvor beschriebenen erfindungsgemäßen Copolymere insbesondere als Zusatzstoffe für Haar- und Hautkösmetika.

Vorzugsweise liegen die erfindungsgemäßen Zubereitungen, die die erfindungsgemäßen Copolymere enthalten, in Form eines Sprays; Gels, Schaums, einer Salbe, Creme, Emulsion, Suspension, Lotion, Milch oder Paste vor. Gewünschtenfalls können auch Liposomen oder Mikrosphären eingesetzt werden.

Vorzugsweise enthalten die erfindungsgemäßen kosmetischen Mittel wenigstens ein erfindungsgemäßes Copolymer, wenigstens einen wie vorstehend definierten Träger B und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Die erfindungsgemäßen Zubereitungen besitzen bevorzugt einen pH-Wert von 2,0 bis 9,3. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.%, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.%.

In einer bevorzugten Ausführungsform der Erfindung weisen die erfindungsgemäßen Zubereitungen einen Anteil an flüchtigen organischen Komponenten von höchstens 80 Gew.-%, bevorzugt höchstens 55 Gew.-% und insbesondere höchstens 35 Gew.-% auf. Ein bevorzugter Gegenstand sind somit kosmetische, bevorzugt haarkosmetische Zubereitungen, die dem low-VOC-Standard, also VOC-80- bzw. VOC-55-Standard entsprechen.

Bevorzugt ist die Verwendung der erfindungsgemäßen Copolymere insbesondere in Haarsprayzubereitungen, welche die folgenden Bestandteile enthalten:

- teilweise oder vollständig neutralisiertes erfindungsgemäßes Copolymer;

- Wasser;

- kosmetisch übliches organisches Lösungsmittel wie beispielsweise Ethanol, Isopropanol und Dimethoxymethan, daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische;

- kosmetisch übliches Treibmittel wie beispielsweise n-Propan, iso-Propan, n-Butan, i-Butan, 2,2-Dimethylbutan, n-Pentan, Isopentan, Dimethylether, Difluorethan, Fluortrichlormethan, Dichlordifluormethan oder Dichlortetrafluorethan, HFC-152 A (1,1-Difluorethan), HFC-134a (1,1,2,2-Tetrafluorethan), $N_2$, $N_2O$ und CO oder deren Gemische.

Zur Neutralisation der erfindungsgemäßen Copolymere und zur Einstellung des pH-Werts der kosmetischen, bevorzugt haarkosmetischen Zubereitungen werden vorteilhaft Alkanolamine eingesetzt. Beispiele (INCI) sind Aminomethylpropanol, Diethanolamin, Diisopropanolamin, Ethanolamin, Methylethanolamin, N-Lauryl-Diethanolamin, Triethanolamin und Triisoproanolamin. Es können sowohl primäre Aminogruppen tragende als auch sekundäre Aminogruppen tragende Alkanolamine verwendet werden.

Außerdem können Alkalihydroxide (z.B. NaOH, bevorzugt KOH) und andere Basen zur Neutralisation verwendet werden (z.B. Histidin, Arginin, Lysin oder Ethylenediamine, Diethylentriamin, Melamin, Benzoguanamin). Alle angegebenen Basen können allein oder als Gemisch mit anderen Basen zur Neutralisation säurehaltiger kosmetischer Produkte eingesetzt werden.

In einer bevorzugten Ausführungsform der Erfindung werden zur Neutralisation Hydroxygruppen enthaltende Amine aus der Gruppe bestehend aus N,N-Dimethylethanolamin, N-Methyldiethanolamin, Triethanolamin, 2-Amino-2-Methylpropanol und Mischungen daraus ausgewählt.

Dabei können sekundäre oder tertiäre Aminogruppen tragende Alkanolamine vorteilhafte Wirkungen zeigen.

Ein Gegenstand der vorliegenden Erfindung sind demnach wässrige kosmetische, bevorzugt haut- und/oder haarkosmetische Zubereitungen die neben dem wenigstens einen erfindungsgemäßen Copolymer und dem Träger B mindestens noch einen Wirk-oder Zusatzstoff ausgewählt aus der Gruppe bestehend aus viskositätsmodifizierenden Stoffen, haarpflegenden Stoffen, haarfestigenden Stoffen, Silikonverbindungen, Lichtschutzstoffen, Fetten, Ölen, Wachsen, Konservierungsmitteln, Pigmenten, löslichen Farbstoffen, partikelförmigen Stoffen und Tensiden enthalten.

Erfindungsgemäße haarkosmetische Formulierungen enthalten in einer bevorzugten Ausführungsform

i) 0,05 bis 20 Gew.-% wenigstens eines erfindungsgemäßen Copolymers,
ii) 20 bis 99,95 Gew.-% Wasser und/oder Alkohol,
iii) 0 bis 50 Gew.-% wenigstens eines Treibgases,
iv) 0 bis 5 Gew.-% wenigstens eines Emulgators,
v) 0 bis 3 Gew.-% wenigstens eines Verdickers, sowie
vi) bis zu 25 Gew.-% weitere Bestandteile.

Unter Alkohol sind alle vorgenannten, in der Kosmetik üblichen Alkohole zu verstehen, z. B. Ethanol, Isopropanol, n-Propanol.

Treibmittel (Treibgase)

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die erfindungsgemäßen kosmetischen, bevorzugt haarkosmetischen Zubereitungen eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Eine wasserhaltige Standard-Aerosol-Sprayformulierung umfasst beispielsweise folgende Bestandteile:

- zu 100 % neutralisiertes erfindungsgemäßes Copolymer
- Alkohol
- Wasser
- Dimethylether und/oder Propan/ n-Butan und/oder Propan/iso-Butan,

Dabei beträgt die Gesamtmenge der flüchtigen organischen Komponenten bevorzugt höchstens 80, besonders bevorzugt höchstens 55 Gew.-% der Zubereitung.

Vorzugsweise enthalten die erfindungsgemäßen kosmetischen, bevorzugt haarkosmetischen Zubereitungen wenigstens ein erfindungsgemäßes Copolymer, wenigstens einen wie vorstehend definierten kosmetisch akzeptablen Träger B und wenigstens einen weiteren, davon verschiedenen Wirk- oder Zusatzstoff, der ausgewählt ist unter kosmetisch aktiven Wirkstoffen, Emulgatoren, Tensiden, Konservierungsmitteln, Parfümölen, Verdickern, Haarpolymeren, Haarconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten,

Konsistenzgebern, Feuchthaltemitteln, Rückfettern, Collagen, Eiweisshydrolysaten, Lipiden, Antioxidantien, Entschäumern, Antistatika, Emollentien, Lanolin-Komponenten, Proteinhydrolysaten und Weichmachern.

Weitere Polymere

Zur gezielten Einstellung der Eigenschaften von kosmetischen, bevorzugt haarkosmetischen Zubereitungen ist es häufig von Vorteil, die erfindungsgemäßen Copolymere in Mischung mit weiteren (haar)kosmetisch üblichen Polymeren einzusetzen.
In einer weiteren bevorzugten Ausführungsform enthält das erfindungsgemäße Mittel 0,01 bis 15 Gew.%, vorzugsweise 0,5 bis 10 Gew.% mindestens eines weiteren synthetischen oder natürlichen nichtionischen, bevorzugt eines filmbildenden Polymers. Unter natürlichen Polymeren werden auch chemisch modifizierte Polymere natürlichen Ursprungs verstanden. Unter filmbildenden Polymeren werden solche Polymere verstanden, die bei Anwendung in 0,01 bis 5%iger wässriger, alkoholischer oder wässrigalkoholischer Lösung in der Lage sind, auf dem Haar einen Polymerfilm abzuscheiden.
Als solche weiteren üblichen Polymere eignen sich dazu beispielsweise anionische, kationische, amphotere, zwitterionische und neutrale Polymere.
Beispiele für solche weiteren Polymere sind

- Copolymere aus Ethylacrylat und Methacrylsäure
- Copolymere aus N-tert.-Butylacrylamid, Ethylacrylat und Acrylsäure
- Polyvinylpyrrolidone
- Polyvinylcaprolactame
- Polyurethane
- Copolymere aus Acrylsäure, Methylmethacrylat, Octylacrylamid, Butylaminoethylmethylacrylat und Hydroxypropylmethacrylat,
- Copolymere aus Vinylacetat und Crotonsäure und/oder (Vinyl)-Neodecanoat,
- Copolymere aus Vinylacetat und/oder Vinylpropionat und N-Vinylpyrrolidon,
- carboxyfunktionelle Copolymere aus Vinylpyrrolidon, t-Butylacrylat, Methacrylsäure,
- Copolymere aus tert.-Butylacrylat, Methacrylsäure und Dimethicone Copolyol.

Überraschenderweise wurde gefunden, dass kosmetische und bevorzugt haarkosmetische Zubereitungen, welche die Polymere A in Kombination mit weiteren Polymeren enthalten, unerwartete Eigenschaften aufweisen. Die erfindungsgemäßen kosmetischen und bevorzugt haarkosmetischen Zubereitungen sind insbesondere hinsichtlich der Gesamtheit ihrer kosmetischen Eigenschaften den Zubereitungen aus dem Stand der Technik überlegen.
Copolymere aus Ethylacrylat und Methacrylsäure (INCI Bezeichnung: Acrylates Copolymer), sind beispielsweise als Handelsprodukte Luviflex®Soft (BASF) erhältlich.
Copolymere aus N-tert.-Butylacrylamid, Ethylacrylat und Acrylsäure (INCI Bezeichnung: Acrylates/Acrylamide Copolymer) sind beispielsweise als Handelsprodukte Ultrahold Strong®, Ultrahold 8® (BASF) erhältlich.
Polyvinylpyrrolidone (INCI Bezeichnung: PVP) sind beispielsweise unter den Handelsnamen Luviskol®K, Luviskol®K30 (BASF) und PVP K® (ISP) erhältlich.
Polyvinylcaprolactame (INCI Polyvinylcaprolactame) sind beispielsweise unter dem Handelsnamen Luviskol Plus® (BASF) erhältlich.
Polyurethane (INCI: Polyurethane -1) sind beispielsweise unter dem Handelsnamen Luviset®PUR erhältlich.
Copolymere aus Acrylsäure, Methylmethacrylat, Octylacrylamid, Butylaminoethylmethylacrylat, Hydroxypropylmethacrylat (INCI: Octylacrylamide/Acrylates/ Butylaminoethyl Methacrylate Copolymer) sind beispielsweise unter den Handelsnamen Amphomer®28-4910 und Amphomer®LV-71 (National Starch) bekannt.
Copolymere aus Vinylacetat und Crotonsäure (INCI: VA/Crotonate/Copolymer) sind beispielsweise unter den Handelsnamen Luviset®CA 66 (BASF), Resyn®28-1310 (National Starch), Gafset® (GAF) oder Aristoflex®A (Celanese) erhältlich.

Copolymere aus Vinylacetat, Crotonsäure und (Vinyl)neodecanoate (INCI: VA/Crotonates/Neodecanoate Copolymer) sind beispielsweise unter den Handelsnamen Resyn®28-2930 (National Starch) und Luviset®CAN (BASF) erhältlich.

Copolymere aus Vinylacetat und N-Vinylpyrrolidon (INCI: PVP/VA) sind beispielsweise unter den Handelsnamen Luviskol VA® (BASF) und PVP/VA (ISP) erhältlich.

Carboxyfunktionelle Copolymere aus Vinylpyrrolidon, t-Butylacrylat, Methacrylsäure sind beipielsweise unter dem Handelsnamen Luviskol®VBM (BASF) erhältlich.

Copolymere aus tert.-Butylacrylat, Methacrylsäure und Dimethicone Copolyol sind beipielsweise unter dem Handelsnamen Luviflex®Silk (BASF) erhältlich.

Geeignete anionische Polymere sind von den Polymeren A verschiedene Homo- und Copolymerisate von Acrylsäure und Methacrylsäure oder deren Salzen, Copolymere von Acrylsäure und Acrylamid und deren Salzen, Natriumsalze von Polyhydroxycarbonsäuren, Copolymere von Acrylsäure und Methacrylsäure mit beispielsweise hydrophoben Monomeren, z.B. $C_4$-$C_{30}$-Alkylester der (Meth)acrylsäure, $C_4$-$C_{30}$-Alkylvinylester, $C_4$-$C_{30}$-Alkylvinylether und Hyaluronsäure so wie weitere unter den Handelsnamen Amerhold®DR-25, Ultrahold®, Luviset®P.U.R., Acronal®, Acudyne®, Lovocryl®, Versatyl®, Amphomer® (28-4910, LV-71), Placise®L53, Gantrez®ES 425, Advantage Plus®, Omnirez®2000, Resyn®28-1310, Resyn®28-2930, Balance®(0/55), Acudyne®255, Aristoflex®A oder Eastman AQ® bekannte Polymere.

Weiterhin umfasst die Gruppe der geeigneten Polymere beispielhaft Balance®CR (National Starch), Balance®47 (National Starch; Octylacrylamid/Acrylate/Butylaminoethylmethacrylate-Copolymer), Aquaflex®FX 64 (ISP; Isobutylen/Ethylmaleimid/ Hydroxyethylmaleimid-Copolymer), Aquaflex®SF-40 (ISP / National Starch; VP/Vinyl Caprolactam/DMAPA Acrylate Copolymer), Allianz®LT-120 (ISP / Rohm & Haas; Acrylat/C1-2 Succinat/Hydroxyacrylat Copolymer), Aquarez® HS (Eastman; Polyester-1).

Geeignet sind auch die Polymere unter den Handelsnamen Diaformer®Z-400 (Clariant; Methacryloylethylbetain/Methacrylat-Copolymer), Diaformer®Z-711 (Clariant; Methacryloylethyl N-oxid/Methacrylat-Copolymer), Diaformer®Z-712 (Clariant; Methacryloylethyl N-oxide/Methacrylat-Copolymer), Omnirez®2000 (ISP; Monoethylester von Poly (Methylvinylether/ Maleinsäure in Ethanol), Amphomer®HC (National Starch; Acrylat/Octylacrylamid-Copolymer), Amphomer®28-4910 (National Starch; Octylacrylamid/Acrylat/ Butylaminoethylmethacrylat-Copolymer), Advantage®HC 37 (ISP; Terpolymer aus Vinylcaprolactam/Vinylpyrrolidon/Dimethylaminoethylmethacrylat), Advantage®LC55 und LC80 oder LC A und LC E, Advantage®Plus (ISP; VA/Butyl Maleate/Isobornyl Acrylate Copolymer), Aculyne®258 (Rohm & Haas; Acrylat/ Hydroxyesteracrylat-Copolymer), Luviset®P.U.R. (BASF, Polyurethane-1), Eastman®AQ 48 (Eastman), Styleze®CC-10 (ISP; VP/DMAPA Acrylates Copolymer), Styleze® 2000 (ISP; VP/Acrylates/Lauryl Methacrylate Copolymer), DynamX® (National Starch; Polyurethane-14 AMP-Acrylates Copolymer), Resyn®XP (National Starch; Acrylates/ Octylacrylamide Copolymer), Fixomer® A-30 (Ondeo Nalco; Polymethacrylsäure (und)Acrylamidomethylpropansulfonsäure), Fixate® G-100 (Noveon; AMP-Acrylates/Allyl Methacrylate Copolymer).

Geeignete Polymere sind auch Copolymere aus (Meth)acrylsäure und Polyetheracrylaten, wobei die Polyetherkette mit einem $C_8$-$C_{30}$-Alkylrest terminiert ist. Dazu zählen z.B. Acrylat/Beheneth-25-methacrylat-Copolymere, die unter der Bezeichnung Aculyn® (Rohm + Haas) erhältlich sind. Besonders geeignete Polymere sind weiterhin Copolymere aus t-Butylacrylat, Ethylacrylat, Methacrylsäure (z.B. Luvimer®100P, Luvimer®Pro55) und Copolymere aus Ethylacrylat und Methacrylsäure (z.B. Luvimer®MAE).

Geeignet sind auch vernetzte Polymere der Acrylsäure, wie sie unter der INCI-Bezeichnung Carbomer erhältlich sind. Derartige vernetzte Homopolymere der Acrylsäure sind kommerziell beispielsweise als Carbopol® (Noveon) erhältlich. Bevorzugt sind auch hydrophob modifizierte vernetzte Polyacrylat-Polymere, wie Carbopol®Ultrez 21 (Noveon). Solche weiteren Polymere können auch zur Rheologiemodifizierung der Zubereitungen, also als Verdicker eingesetzt werden.

Als zusätzliche Polymere weiterhin geeignet sind wasserlösliche oder wasserdispergierbare Polyester, Polyharnstoffe, Polyurethane, Polyurethanharnstoffe, gegebenenfalls mit Alkoholen umgesetzte Maleinsäureanhydridcopolymere oder anionische Polysiloxane.

Weiterhin zur Verwendung gemeinsam mit den Polymeren A geeignete Polymere sind z.B. auch kationische und kationogene Polymere. Dazu zählen beispielsweise

- Copolymere aus N-Vinylpyrrolidon/N-Vinylimidazoliumsalzen (erhältlich beispielsweise unter den Handelsnamen Luviquat®FC, Luviquat®HM, Luviquat®MS, Luviquat®Care, Luviquat® UltraCare (BASF),
- Copolymere aus N-Vinylcaprolactam/N-Vinylpyrrolidon/N-Vinylimidazoliumsalzen (erhältlich beispielsweise unter dem Handelsnamen Luviquat®Hold),
- Copolymere aus N-Vinylpyrrolidon/Dimethylaminoethylmethacrylat, quaternisiert mit Diethylsulfat (erhältlich beispielsweise unter dem Handelsnamen Luviquat®PQ11),
- Copolymere aus Vinylpyrrolidon, Methacrylamid, Vinylimidazol (Luviset®Clear)
- kationische Cellulosederivate (Polyquaternium-4 und -10),

- Acrylamidcopolymere (Polyquaternium-7),
- Guar-hydroxypropyltrimethylammoniumchlorid (INCI: Hydroxypropyl Guar Hydroxypropyltrimonium Chloride),
- Polyethylenimine und deren Salze,
- Polyvinylamine und deren Salze,
- Polymere auf Basis von Dimethyldiallylammoniumchlorid (Merquat®),
- Polymere, die durch Reaktion von Polyvinylpyrrolidon mit quaternären Ammoniumverbindungen entstehen (Gafquat®),
- Hydroxyethylcellulose mit kationischen Gruppen (Polymer®JR) und
- kationische Polymere auf pflanzlicher Basis, z.B. Guarpolymere, wie die Jaguar®-Marken der Fa. Rhodia.

Als weitere Haarkosmetik-Polymere sind auch neutrale Polymere geeignet wie

- Polyvinylpyrrolidone,
- Copolymere aus N-Vinylpyrrolidon und Vinylacetat und/oder Vinylpropionat,
- Polysiloxane,
- Polyvinylcaprolactame und
- Copolymere mit N-Vinylpyrrolidon,
- Cellulosederivate,
- Polyasparaginsäuresalze und Derivate,
- Polyamide, z.B. auf Basis von Itaconsäure und aliphatischen Diaminen, wie in der DE-A-43 33 238 beschrieben,

Zu den vorgenannten Polymerarten gehören die unter den Handelsnamen bekannten Luviskol® (K, VA, Plus), PVP K, PVP/VA, Advantage®HC, Luviflex®Swing, Kollicoat®IR, $H_2$OLD®EP-1.

Außerdem geeignet als weitere Polymere sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaluronsäure- und RNA-Derivate.

Geeignete Mischungspartner für die erfindungsgemäßen Polymere sind auch zwitterionische Polymere, wie sie beispielsweise in den deutschen Patentanmeldungen DE 39 29 973, DE 21 50 557, DE 28 17 369 und DE 37 08 451 offenbart sind sowie Methacroylethylbetain/Methacrylat-Copolymere, die unter der Bezeichnung Amersette® (Amerchol) im Handel erhältlich sind oder Copolymere aus Hydroxyethylmethacrylat, Methylmethacrylat, N,N-Dimethylaminoethylmethacrylat und Acrylsäure (Jordapon®).

Weitere geeignete Polymere sind auch betainische Polymere wie Yukaformer (R205, SM) und Diaformer.

Als Mischungspartner geeignete Polymere sind auch nichtionische, siloxanhaltige, wasserlösliche oder -dispergierbare Polymere, z.B. Polyethersiloxane, wie Tegopren® (Goldschmidt) oder Belsil® (Wacker).

Kosmetisch und/oder dermatologisch aktive Wirkstoffe

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Bevorzugte kosmetische Pflege- und Wirkstoffe sind AHA-Säuren, Fruchtsäuren, Ceramide, Phytantriol, Collagen, Vitamine und Provitamine, beispielsweise Vitamin A, E und C, Retinol, Bisabolol und Panthenol. Besonders bevorzugt als kosmetischer Pflegestoff in den erfindungsgemäßen Zubereitungen ist Panthenol, das beispielsweise als D-Panthenol®USP, D-Panthenol®50 P, D-Panthenol®75 W, D,L-Panthenol®50 W kommerziell erhältlich ist.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt.

Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc..

Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd,

Sorbinsäure, Benzoesäure, Salicylsäure, etc.. Derartige desodorierend wirkende Stoffe sind z. B. Zinkricinoleat, Triclosan, Undecylensäurealkylolamide, Citronensäuretriethylester, Chlorhexidin etc.. Die erfindungsmäßen Zubereitungen enthalten bevorzugt 0,01 bis 5, besonders bevorzugt 0,05 bis 1 Gew.% mindestens eines Konservierungsmittels.

Geeignete weitere Konservierungsmittel sind die im International Cosmetic Ingredient Dictionary and Handbook, 9. Auflage mit der Funktion "Preservatives" aufgeführten Stoffe, z.B . Phenoxyethanol, Benzylparaben, Butylparaben, Ethylparaben, Isobutylparaben, Isopropylparaben, Methylparaben, Propylparaben, Iodopropinylbutylcarbamat, Methyldibromoglutaronitril, DMDM Hydantoin.

UV-Filtersubstanzen

Die erfindungsgemäßen Zubereitungen können in einer Ausführungsform öllösliche und/oder wasserlösliche UVA- und/oder UVB-Filter enthalten.

Die Gesamtmenge der Filtersubstanzen beträgt vorzugsweise 0,01 bis 10 Gew.% oder von 0,1 bis 5 Gew.%, besonders bevorzugt von 0,2 bis 2 Gew.%, bezogen auf das Gesamtgewicht der Zubereitungen.

Der größte Teil der Lichtschutzmittel in den zum Schutz der menschlichen Epidermis dienenden Zubereitungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Die UVB-Filter können öllöslich oder wasserlöslich sein. Vorteilhafte UVB-Filtersubstanzen sind z.B.:

i) Benzimidazolsulfonsäure-Derivate wie z.B. 2-Phenylbenzimidazol-5-sulfonsäure und deren Salze;
ii) Benzotriazol-Derivate wie z.B. 2,2'-Methylen-bis-(6-(2H-benzotriazol-2-yl)-4-(1,1,3,3-tetramethylbutyl)-phenol);
iii) 4-Aminobenzoesäure-Derivate, vorzugsweise 4-(Dimethylamino)-benzoesäure(2-ethylhexyl)ester, 4-(Dimethy1amino)benzoesäureamylester;
iv) Ester der Benzalmalonsäure, vorzugsweise 4-Methoxybenzalmalonsäuredi(2-ethylhexyl)ester;
v) Ester der Zimtsäure, vorzugsweise 4-Methoxyzimtsäure(2-ethylhexyl)ester, 4-Methoxyzimtsäureisopentylester;
vi) Derivate des Benzophenons, vorzugsweise 2-Hydroxy-4-methoxybenzophenon, 2-Hydroxy-4-methoxy-4'-methyl benzophenon, 2,2'-Dihydroxy-4-methoxybenzophenon;
vii) Methylidencampherderivate, vorzugsweise 4-Methylbenzylidencampher, Benzyl idencampher;
viii) Triazinderivate, vorzugsweise 4,4',4"-(1,3,5-Triazin-2,4,6-triylimino)-tris-benzoesäure-tris-(2-ethylhexylester) [INCI: Diethylhexyl Butamido Triazine, UVA-Sorb® HEB (Sigma 3V)] und 2,4,6-Tris-[anilino-(p-carbo-2'-ethyl-1'-hexyloxy)]-I ,3,5-triazin [INCI: Octyl Triazone, Uvinul®T 150 (BASF)].

Vorteilhaft zu verwendende wasserlösliche UVB-Filtersubstanzen sind z.B. Sulfonsäure-Derivate des 3-Benzylidencamphers, wie z.B. 4-(2-Oxo-3-bornylidenmethyl) benzolsulfonsäure, 2-Methyl-5-(2-oxo-3-bornylidenmethyl)sulfonsäure und deren Salze.

Vorteilhaft zu verwendende UVA-Filter sind z.B.:

- 1,4-Phenylendimethincamphersulfonsäurederivate wie z.B. 3,3'-( 1,4-phenylendimethin)-bis-(7,7-dimethyl-2-oxobicyclo[2.2.1]-heptan-I - methamsulfonsäure und ihre Salze
- 1,3,5-Triazinderivate wie 2,4-Bis{[(2-ethylhexyloxy)-2-hydroxy)-phenyl}-6-(4-methoxyp henyl)-I ,3,5)-triazin (z.B. Tinosorb®S (Ciba))
- Dibenzoylmethanderivate, vorzugsweise 4-Isopropyldibenzoylmethan, 4-(tert.-Butyl)-4'-methoxydibenzoylmethan
- Benzoxazol-Derivate, beispielsweise das 2,4-Bis-[4-[5-(1,1-dimethylpropyl)benzoxazol-2-yl]phenylimino]-6-[(2-ethylexyl)imino]-1,3,5- triazin (CAS Nr. 288254-1 6-0, Uvasorb®K2A (3V Sigma))
- Hydroxybenzophenone, beispielsweise der 2-(4'-Diethylamino-2'-hydoxybenzoyl)-benzoesäurehexylester (auch: Aminobenzophenon) (Uvinul®A Plus (BASF))

Ferner kann erfindungsgemäß gegebenenfalls von Vorteil sein, Zubereitungen mit weiteren UVA- und/oder UVB-Filtern zu versehen, beispielsweise bestimmten Salicylsäurederivaten wie 4-Isopropylbenzylsalicylat, 2-Ethylhexylsalicylat, Octylsalicylat, Homomenthylsalicylat. Die Gesamtmenge an Salicylsäurederivaten in den kosmetischen Zubereitungen wird vorteilhaft aus dem Bereich von 0,1-15,0, bevorzugt 0,3-10,0 Gew.-% gewählt, bezogen auf das Gesamtgewicht der Zubereitungen. Ein weiterer erfindungsgemäß vorteilhaft zu verwendender Lichtschutzfilter ist Ethylhexyl-2-cyano-3,3-diphenylacrylat (Octocrylen, Uvinul®N 539 (BASF)).

Die folgende Tabelle stellt beispielhaft einige für die Verwendung in den erfindungsgemäßen Zubereitungen geeignete Lichtschutzfilter zusammen:

| Nr. | Stoff | CAS-Nr. |
|---|---|---|
| 1 | 4-Ami nobenzoesäure | 150-13-0 |

(fortgesetzt)

| Nr. | Stoff | CAS-Nr. |
|---|---|---|
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy] disiloxanyl]propyl]phenol | 155633-54-8 |

(fortgesetzt)

| Nr. | Stoff | CAS-Nr. |
|---|---|---|
| 38 | 1,1-[(2,2'-Dimethylpropoxy)carbonyl]-4,4-diphenyl-1,3-butadien | 363602-15-7 |

Geeignete UV-Lichtschutzfilter mit der CAS-Nr. 113010-52-9 sind beispielsweise unter der Bezeichnung Uvinul®P 25 kommerziell erhältlich.

Auch polymere oder polymergebundene Filtersubstanzen können erfindungsgemäß verwendet werden.

Metalloxide wie Titandioxid oder Zinkoxid können ebenfalls vorteilhaft zum Schutz vor schädlicher Sonnenstrahlung eingesetzt werden. Ihre Wirkung beruht im wesentlichen auf Reflexion, Streuung und Absorption der schädigenden UV-Strahlung und hängt wesentlich von der Primärpartikelgröße der Metalloxide ab. Die erfindungsgemäßen kosmetischen Zubereitungen können vorteilhafterweise außerdem anorganische Pigmente auf Basis von Metalloxiden und/oder anderen in Wasser schwerlöslichen oder unlöslichen Metallverbindungen, ausgewählt aus der Gruppe der Oxide des Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden enthalten. Besonders bevorzugt handelt es sich um Pigmente auf der Basis ZnO.

Die anorganischen Pigmente können dabei in gecoateter Form vorliegen, d.h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.

Zur Verwendung in den erfindungsgemäßen Zubereitungen geeignete Lichtschutzmittel sind die in der EP-A 1 084 696 in den Absätzen [0036] bis [0053] genannten Verbindungen, worauf an dieser Stelle vollumfänglich Bezug genommen wird. Für die erfindungsgemäße Verwendung geeignet sind alle UV-Lichtschutzfilter, die in Anlage 7 (zu § 3b) der deutschen Kosmetik-Verordnung unter "Ultraviolett-Filter für kosmetische Mittel" genannt sind.

Die Aufzählung der genannten UV-Lichtschutz-Filter, die in den erfindungsgemäßen Zubereitungen verwendet werden können, ist nicht abschließend.

Verdickungsmittel

Geeignete Verdickungsmittel sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird.

Konsistenzregulatoren ermöglichen die Einstellung der gewünschten Viskosität von beispielsweise Shampoos. Verdicker, die durch eine Vergrößerung der Tensidmicellen bzw. durch eine Quellung der Wasserphase viskositätsaufbauend wirken, entstammen chemischsehr unterschiedlichen Stoffklassen.

Geeignete Verdickungsmittel für die erfindungsgemäßen Zubereitungen sind beispielsweise vernetzte Polyacrylsäuren und deren Derivate, Polysaccharide wie Xanthan-gum, Guar-Guar, Agar-Agar, Alginate oder Tylosen, Cellulosederivate, z. B. Carboxymethylcellulose oder Hydroxycarboxymethylcellulose, ferner höhermolekulare Polyethylenglycolmono- und -diester von Fettsäuren, Fettalkohole, Monoglyceride und Fettsäuren, Polyvinylalkohol und Polyvinylpyrrolidon.

Geeignete Verdicker sind kommerziell erhältlich unter den Handelsnamen Carbopol® (Noveon), Ultrez® (Noveon), Luvigel® EM (BASF), Capigel®98 (Seppic), Synthalene® (Sigma), Aculyn® (Rohm und Haas) wie Aculyn® 22 (Copolymerisat aus Acrylaten und Methacrylsäureethoxylaten mit Stearylrest (20 EO-Einheiten)) und Aculyn® 28 (Copolymerisat aus Acrylaten und Methacrylsäureethoxilaten mit Behenylrest (25 EO-Einheiten)).

Geeignete Verdickungsmittel sind weiterhin beispielsweise Aerosil-Typen (hydrophile Kieselsäuren), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Hornologenverteilung oder Alkyloligoglucoside sowie Elektrolyte wie Kochsalz und Ammoniumchlorid.

Besonders bevorzugte Verdickungsmittel zur Herstellung von Gelen sind Ultrez®21, Aculyn®28, Luvigel® EM und Capigel®98.

Insbesondere bei höher konzentrierten Shampoo-Formulierungen können zur Regulierung der Konsistenz auch Stoffe zugesetzt werden, die die Viskosität der Formulierungverringern, wie z. B. Propylenglykol oder Glycerin. Diese Stoffe beeinflussen die Produkteigenschaften nur wenig.

Gelbildner

Wird für die erfindungsgemäßen Zubereitungen der Einsatz von Gelbildnern gewünscht, so können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen,

Polysaccharide, z.B. Xanthum Gummi, Caprylic/Capric Triglyceride, Sodium

acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Emulgatoren

Als Emulgatoren kommen beispielsweise nichtionogene Tenside aus mindestens einer der folgenden Gruppen in Frage:

i) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;

ii) C12/18-Fettsäuremono- und -diester von Anlagerungsprodukten von 1 bis 30 Mol Ethylenoxid an Glycerin;

iii) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;

iv) Alkylmono- und -oligoglycoside mit 8 bis 22 Kohlenstoffatomen im Alkylrest und deren ethoxylierte Analoga;

v) Anlagerungsprodukte von 15 bis 60 Mol Ethylenoxid an Ricinusöl und/oder gehärtetes Ricinusöl;

vi) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;

vii) Anlagerungsprodukte von 2 bis 15 Mol Ethylenoxid an Ricinusöl und/oder gehärtetes Ricinusöl;

viii) Partialester auf Basis linearer, verzweigter, ungesättigter bzw. gesättigter $C_6/_{22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);

ix) Mono-, Di- und Trialkylphosphate sowie Mono-, Di- und/oder Tri-PEGalkylphosphate und deren Salze;

x) Wollwachsalkohole;

xi) Polysiloxan-Polyalkyl-Polyether-Copolymere bzw. entsprechende Derivate;

xii) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie

xiii) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und - diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12}$ bis $C_{18}$-Fettsäuremono- und -diester von Anlagerungsprodukten von Ethylenoxid an Glycerin sind aus DE-PS 2024051 als Rückfettungsmittel für kosmetische Zubereitungen bekannt. $C_8$ bis $C_{18}$-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cyclischer Zuckerrest glycosidisch an den Fettalkohol gebunden ist, als auch coligomere Glycoside mit einem Oligomerisationsgrad bis vorzugsweise etwa 8 geeignet sind. Der Oligomerisierungsgrad ist dabei ein statistischer Mittelwert, dem eine für solche

technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und/oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxy-methylglycinat.

Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_8$ bis $C_{18}$-Alkyl- oder -Acylgruppe im Molekül mindestens eine freie Aminogruppe und mindestens eine -COOH- und/oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12}$ bis $C_{18}$-Acylsarcosin.

Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Antioxidantien

Ein zusätzlicher Gehalt der Zubereitungen an Antioxidantien kann von Vorteil sein. Erfindungsgemäß können als Antioxidantien alle für kosmetische Anwendungen geeigneten oder gebräuchlichen Antioxidantien verwendet werden. Vorteilhaft werden die Antioxidantien gewählt aus der Gruppe bestehend aus Aminosäuren (z.B. Glycin, Histidin, Tyrosin, Tryptophan) und deren Derivaten, Imidazolen (z.B. Urocaninsäure) und deren Derivaten, Peptiden wie D,L-Carnosin, D-Carnosin, L-Carnosin und deren Derivaten (z.B. Anserin), Carotinoide, Carotinen (z.B. α-Carotin, β-Carotin, γ-Lycopin) und deren Derivaten, Chlorogensäure und deren Derivaten, Liponsäure und deren Derivaten (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Coniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol , Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist es vorteilhaft, diese in Konzentrationen von 0,001 bis 10 Gew.-%, bezogen auf das Gesamtgewicht der Zubereitung, bereitzustellen.

Sofern Vitamin A, bzw. Vitamin-A-Derivate, bzw. Carotine bzw. deren Derivate das oder die Antioxidantien darstellen, ist vorteilhaft, diese in Konzentrationen von 0,001 bis 10 Gew.-%, bezogen auf das Gesamtgewicht der Zubereitung, bereitzustellen.

Parfümöle

Die kosmetischen, bevorzugt haarkosmetischen Zubereitungen können Parfümöle enthalten. Als Parfümöle seien beispielsweise Gemische aus natürlichen und synthetischen Riechstoffen genannt. Natürliche Riechstoffe sind

Extrakte von Blüten (Lilie, Lavendel, Rose, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, 4-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonat, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terioneol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzeöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexyl-salicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen eingesetzt.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Silikonverbindungen

In einer Ausführungsform enthalten die erfindungsgemäßen Zubereitungen als haarpflegenden Zusatzstoff mindestens eine Silikonverbindung in einer Menge von vorzugsweise 0,01 bis 15 Gew.%, besonders bevorzugt von 0,1 bis 5 Gew.%. Die Silikonverbindungen umfassen flüchtige und nicht-flüchtige Silikone und in dem Mittel lösliche und unlösliche Silikone. Bei einer Ausführungsform handelt es sich um hochmolekulare Silikone mit einer Viskosität von 1.000 bis 2.000.000 cSt bei 25°C, vorzugsweise 10.000 bis 1.800.000 oder 100.000 bis 1.500.000. Die Silikonverbindungen umfassen Polyalkyl- und Polyarylsiloxane, insbesondere mit Methyl-, Ethyl-, Propyl-, Phenyl-, Methylphenyl- und Phenylmethylgruppen. Bevorzugt sind Polydimethylsiloxane, Polydiethylsiloxane, Polymethylphenylsiloxane. Bevorzugt sind auch glanzgebende, arylierte Silikone mit einem Brechungsindex von mindestens 1,46, oder mindestens 1,52. Die Silikonverbindungen umfassen insbesondere die Stoffe mit den INCI-Bezeichnungen Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, Dimethicone Crosspolymer. Geeignet sind auch Silikonharze und Silikonelastomere, wobei es sich um hochvernetzte Siloxane handelt.

Bevorzugte Silikone sind cyclische Dimethylsiloxane, lineare Polydimethylsiloxane, Blockpolymere aus Polydimethylsiloxan und Polyethylenoxid und/oder Polypropylenoxid, Polydimethylsiloxane mit end- oder seitenständigen Polyethylenoxid- oder Polypropylenoxidresten, Polydimethylsiloxane mit endständigen Hydroxylgruppen, phenylsubstituierte Polydimethylsiloxane, Silikonemulsionen, Silkonelastomere, Silikonwachse, Silikongums und aminosubstituierte Silikone (CTFA: Amodimethicone).

Haarkonditioniermittel

In einer Ausführungsform enthalten die erfindungsgemäßen Zubereitungen 0,01 bis 20, bevorzugt von 0,05 bis 10, besonders bevorzugt von 0,1 bis 5 Gew.% mindestens eines Konditioniermittels.

Erfindungsgemäß bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humectant, Skin-Conditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen. Weitere vorteilhafte Konditionierungsmittel stellen beispielsweise die nach INCI als Polyquaternium bezeichneten Verbindungen dar (insbesondere Polyquaternium-1 bis Polyquaternium-56).

Zu den geeigneten Konditionierungsmitteln zählen beispielsweise auch polymere quaternäre Ammoniumverbindungen, kationische Cellulosederivate, Chitosanderivate und Polysaccharide.

Das Konditioniermittel ist bevorzugt ausgewählt aus Betain, Panthenol, Panthenylethylether, Sorbitol, Proteinhydrolysaten, Pflanzenextrakten; A-B-Block-Copolymeren aus Alkylacrylaten und Alkylmethacryaten; A-B-Block-Copolymeren aus Alkylmethacrylaten und Acrylnitril; A-B-A-Block-Copolymeren aus Lactid und Ethylenoxid; A-B-A-Block-Copolymeren aus Caprolacton und Ethylenoxid; A-B-C-Block-Copolymeren aus Alkylen- oder Alkadienverbindungen, Styrol und Alkylmethacrylaten; A-B-C-Block-Copolymeren aus Acrylsäure, Styrol und Alkylmethacrylaten, sternförmigen Block-Copolymeren, hyperverzweigten Polymeren, Dendrimeren, intrinsisch elektrisch leitfähigen 3,4-Polyethylendioxythiophenen und intrinsisch elektrisch leitfähigen Polyanilinen.

Weitere erfindungsgemäß vorteilhafte Konditioniermittel stellen Cellulosederivate und quaternisierte Guargum Derivate, insbesondere Guar Hydroxypropylammoniumchlorid (z.B. Jaguar Excel®, Jaguar C 162® (Rhodia), CAS 65497-29-2, CAS 39421-75-5) dar. Auch nichtionische Poly-N-vinylpyrrolidon/Polyvinylacetat-Copolymere (z.B. Luviskol®VA 64 (BASF)), anionische Acrylat-Copolymere (z.B. Luviflex®Soft (BASF)), und/oder amphotere Amid/Acrylat/Methacrylat Copolymere (z.B. Amphomer® (National Starch)) können erfindungsgemäß vorteilhaft als Konditioniermittel eingesetzt werden.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind

i) Glycerin;

ii) Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1000 Dalton;

iii) technische Oligoglyceringemische mit einem Eigenkondensationsgrad von 1,5 bis 10 wie etwa technische Diglyceringemische mit einem Diglyceringehalt von 40 bis 50 Gew.-%;

iv) Methylolverbindungen, wie insbesondere Trimethylolethan, Trimetylolpropan, Trimetylolbutan, Pentaerythrit und Dipentaerythrit;

v) Niedrigalkylglucoside, insbesondere solche mit 1 bis 8 Kohlenstoffen im Alkylrest, wie beispielsweise Methyl- und Butylglucosid;

vi) Zuckeralkohole mit 5 bis 12 Kohlenstoffatomen, wie beispielsweise Sorbit oder Mannit;

vii) Zucker mit 5 bis 12 Kohlenstoffatomen, wie beispielsweise Glucose oder Saccharose;

viii) Aminozucker, wie beispielsweise Glucamin.

Öle, Fette und Wachse

Die erfindungsgemäßen kosmetischen, bevorzugt haarkosmetischen Zubereitungen können auch Öle, Fette oder Wachse enthalten. Solche werde vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen

Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat Dicaprylyl Carbonat (Cetiol CC) und Cocoglyceride (Myritol 331), Butylen Glycol Dicaprylat/Dicaprat und Dibutyl Adipat sowie synthetische, halbsynthetische und natürliche Gemische solcher Ester, wie z.B. Jojobaöl.

Ferner können eine oder mehrere Olkomponenten vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Erfindungsgemäß vorteilhaft wird die Olkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Erfindungsgemäß vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Erfindungsgemäß besonders bevorzugt werden als Öle mit einer Polarität von 5 bis 50 mN/m Fettsäuretriglyceride, insbesondere Sojaöl und/oder Mandelöl eingesetzt.

Ferner kann die Ölphase vorteilhaft gewählt werden aus der Gruppe der Guerbetalkohole. Diese sind selbst bei niederen Temperaturen flüssig und bewirken praktisch keine Hautreizungen. Vorteilhaft können sie als fettende, überfettende und auch rückfettend wirkende Bestandteile in kosmetischen Zusammensetzungen eingesetzt werden.

Die Verwendung von Guerbet-Alkoholen in Kosmetika ist an sich bekannt. Erfindungsgemäß bevorzugte Guerbet-Alkohole sind 2-Butyloctanol (beispielsweise als Isofol®12 (Condea) kommerziell erhältlich) und 2-Hexyldecanol (beispielsweise als Isofol®16 (Condea) kommerziell erhältlich).

Auch Mischungen von erfindungsgemäßen Guerbet-Alkoholen sind erfindungsgemäß vorteilhaft zu verwenden wie beispielsweise Mischungen aus 2-Butyloctanol und 2-Hexyldecanol (beispielsweise als Isofol®14 (Condea) kommerziell erhältlich).

Auch beliebige Abmischungen solcher Öl- und Wachskomponenten sind vorteilhaft im Sinne der vorliegenden Erfindung einzusetzen. Unter den Polyolefinen sind Polydecene die bevorzugten Substanzen.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Vorteilhaft sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise Syncrowax®HRC (Glyceryltribehenat), und Syncrowax®AW 1 C ($C_{18-36}$-Fettsäure) sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Cetyl Ricinoleate wie beispielsweise Tegosoft®CR, Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride wie beispielsweise Hydriertes Soy Glycerid, Trihydroxystearin, Fettsäuren, Fettsäureester und Gly kolester wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan. Erfindungsgemäß können die Fett- und/oder Wachskomponenten sowohl einzeln als auch als Gemisch in den Zusammensetzungen verwendet werden.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, $C_{12-15}$-Alkylbenzoat, Capryl-Caprin-säure-triglycerid, Dicaprylylether. Besonders vorteilhaft sind Mischungen aus Octyldodecanol, Capryl-Caprinsäure-triglycerid, Dicaprylylether, Dicaprylyl Carbonat, Cocoglyceriden oder Mischungen aus $C_{12-15}$-Alkylbenzoat und 2-Ethylhexylisostearat, Mischungen aus $C_{12-15}$-Alkylbenzoat und Butylen Glycol Dicaprylat/Dicaprat sowie Mischungen aus $C_{12-15}$-Alkylbenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw.

Polydecen vorteilhaft im Sinne der vorliegenden Erfindung zu verwenden.

Die Ölkomponente wird ferner vorteilhaft aus der Gruppe der Phospholipide gewählt. Als erfindungsgemäß vorteilhaftes Paraffinöl kann erfindungsgemäß Merkur®Weissoel Pharma 40 von Merkur Vaseline, Shell Ondina® 917, Shell Ondina® 927, Shell

Oil 4222, Shell Ondina®933 von Shell & DEA Oil, Pionier® 6301 S, Pionier® 2071 (Hansen & Rosenthal) eingesetzt werden.

Geeignete kosmetisch verträgliche Öl- und Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier in vollem Umfang Bezug genommen wird.

Der Gehalt an Ölen, Fetten und Wachsen beträgt höchstens 30, bevorzugt 20, weiter bevorzugt höchstens 10 Gewichts-%, bezogen auf das Gesamtgewicht der Zusammensetzung.

Pigmente

In einer Ausführungsform enthalten die erfindungsmäßen Zubereitungen mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb-oder Glanzeffekte am Haar sind vorzugsweise temporär, d.h. sie verbleiben bis zur nächsten Haarwäsche auf dem Haar und können durch Waschen der Haare mit üblichen Shampoos wieder entfernt werden.

Die Pigmente liegen in der Produktmasse in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 µm, insbesondere 3 bis 150 µm, besonders bevorzugt 10 bis 100 µm. Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxid rot, um Glanzpigmente , Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist.

Geeignet sind Metalloxide, -hydroxide und -oxidhydrate, Mischphasenpigmente, schwefelhaltige Silicate, Metallsulfide, komplexe Metallcyanide, Metallsulfate, - Chromate und -molybdate sowie die Metalle selbst (Bronze-Pigmente). Geeignet sind insbesondere Titandioxid (CI 77891), schwarzes Eisenoxid (CI 77499), gelbes Eisenoxid (CI 77492), rotes und braunes Eisenoxid (CI 77491 ), Manganviolett (CI 77742), Ultramarine (Natrium-Aluminiumsulfosilikate, CI 77007, Pigment Blue 29), Chromoxidhydrat (C177289), Eisenblau (Ferric Ferro-Cyanide, CI7751 0), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

In einer Ausführungsform enthalten die erfindungsmäßen Zubereitungen 0,01 bis 10, besonders bevorzugt von 0,05 bis 5 Gew.% mindestens eines partikelförmigen Stoffes. Geeignete Stoffe sind z.B. Stoffe, die bei Raumtemperatur (25°C) fest sind und in Form von Partikeln vorliegen. Geeignet sind etwa Silica, Silikate, Aluminate, Tonerden, Mica, Salze, insbesondere anorganische Metallsalze, Metalloxide, z.B. Titandioxid, Minerale und Polymerpartikel.

Die Partikel liegen in dem Mittel ungelöster, vorzugsweise stabil dispergierter Form vor und können sich nach Aufbringen auf die Anwendungsoberfläche und Verdampfen des Lösungsmittels in fester Form abscheiden.

Bevorzugte partikelförmige Stoffe sind Silica (Kieselgel, Siliciumdioxid) und Metallsalze, insbesondere anorganische Metallsalze, wobei Silica besonders bevorzugt ist. Metallsalze sind z.B. Alkali- oder Erdalkalihalogenide wie Natriumchlorid oder Kaliumchlorid; Alkali- oder Erdalkalisulfate wie Natriumsulfat oder Magnesiumsulfat.

Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc.

Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc.:

Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc..

Geeignete Antiphlogistika, die Hautreizungen entgegenwirken, sind z. B. Allantoin, Bisabolol, Dragosantol, Kamillenextrakt, Panthenol, etc..

Darreichungsform

Die erfindungsgemäßen Zubereitungen sind in einer bevorzugten Ausführungsform versprühbar, beispielsweise als Aerosol- oder Pumpspray-Zubereitung.

Die erfindungsgemäßen Zubereitungen können in verschiedenen Formen angewendet werden, wie beispielsweise als Lotion, als Non-Aerosol Sprühlotion, welches mittels einer mechanischen Vorrichtung zum Versprühen zum Einsatz kommt, als AerosolSpray welches mittels eines Treibmittels versprüht wird, als Aerosol-Schaum oder als Non-Aerosol Schaum, welcher in Kombination mit einer geeigneten mechanischen Vorrichtung zum Verschäumen der Zusammensetzung vorliegt, als Haarcreme, als Haarwachs, als Gel, als Flüssiggel, als versprühbares Gel oder als Schaumgel.

Auch ein Einsatz in Form einer mit einem üblichen Verdicker verdickten Lotion ist möglich.

In einer Ausführungsform liegt das erfindungsgemäße Mittel in Form eines Gels, in

Form einer viskosen Lotion oder in Form eines Sprühgels, welches mit einer mechanischen Vorrichtung versprüht wird, vor und enthält mindestens eines der oben genannten Verdickungsmittel in einer Menge von vorzugsweise 0,05 bis 10, besonders bevorzugt von 0,1 bis 2 Gew.% und weist eine Viskosität von mindestens 250 mPas auf. Die Viskosität des Gels beträgt vorzugsweise von 500 bis 50.000 mPas, besonders bevorzugt von 1.000 bis 15.000 mPas bei 25°C.

In einer anderen Ausführungsform liegt die erfindungsgemäße Zubereitung in Form einer O/W-Emulsion, einer W/O-Emulsion oder einer Mikroemulsion vor und und enthält mindestens eines der oben genannten, in Wasser emulgierten Öle oder Wachse sowie mindestens ein kosmetisch übliches Tensid.

In einer bevorzugten Ausführungsform liegt die erfindungsgemäße Zubereitung in Form eines Sprühproduktes vor, entweder in Kombination mit einer mechanischen Pumpsprühvorrichtung oder in Kombination mit mindestens einem der vorgenannten Treibmittel. Ein bevorzugten Aerosolspray enthält zusätzlich Treibmittel in einer solchen Menge, dass die Gesamtmenge der flüchtigen organischen Komponenten 80, insbesondere 55 Gew.% der Zubereitung nicht übersteigt und wird in einem Druckbehälter abgefüllt.

Ein Non-Aerosol-Haarspray wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem die erfindungsgemäße kosmetische Zubereitung unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer weiteren Ausführungsform liegt die erfindungsgemäße Zubereitung in Form eines verschäumbaren Produktes (Mousse) in Kombination mit einer Vorrichtungen zum Verschäumen vor, enthält mindestens eine übliche, hierfür bekannte schaumgebende Substanz, z.B. mindestens ein schaumbildendes Tensid oder mindestens ein schaumbildendes Polymer. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden. Das Produkt liegt entweder in Kombination mit einer mechanischen Pumpschäumvorrichtung (Pumpschaum) oder in Kombination mit mindestens einem Treibmittel (Aerosol-Schaum) in einer Menge von vorzugsweise 1 bis 20, insbesondere von 2 bis 10 Gew.%, vor. Treibmittel sind z.B. ausgewählt aus Propan, Butan, Dimethylether und fluorierten Kohlenwasserstoffen.

Ein Gegenstand der Erfindung ist somit eine kosmetische, bevorzugt haarkosmetische Zubereitung in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

Das Mittel wird unmittelbar vor der Anwendung verschäumt und als Schaum in das Haar eingearbeitet und kann anschließend ausgespült werden oder ohne Ausspülen im Haar belassen werden.

Eine erfindungsgemäß bevorzugte Formulierung für Aerosolhaarschäume enthält

i) 0,1 bis 10 Gew.-% wenigstens eines erfindungsgemäßen Copolymers,

ii) 55 bis 99,8 Gew.-% Wasser und Alkohol,
iii) 5 bis 20 Gew.-% eines Treibmittels,
iv) 0,1 bis 5 Gew.-% eines Emulgators,
v) 0 bis 10 Gew.-% weitere Bestandteile,

wobei die Gesamtmenge von VOC höchstens 80 und bevorzugt höchstens 55 Gew.-% beträgt.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglykolcetylether; Ceteareth e, z. B. Ceth eareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

i) 0,1 bis 10 Gew.-% erfindungsgemäßes Copolymer,
ii) 80 bis 99,85 Gew.-% Wasser und Alkohol,
iii) 0 bis 3 Gew.-%, bevorzugt 0,05 bis 2 Gew.-% Gelbildner,
iv) 0 bis 20 Gew.-% weitere Bestandteile.

wobei die Gesamtmenge von VOC höchstens 80 und bevorzugt höchstens 55 Gew.-% beträgt.

Bei der Herstellung von Gelen auf Basis der erfindungsgemäßes Copolymer können übliche Gelbildner eingesetzt werden, beispielsweise, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44. Als Gelbildner geeignete vernetzte Homopolymere der Acrylsäure sind beispielsweise kommerziell unter dem Namen Carbopol® (Noveon) erhältlich. Bevorzugt sind auch hydrophob modifizierte vernetzte Polyacrylat Polymere, wie Carbopol®Ultrez 21 (Noveon). Weitere Beispiele für als Gelbildner geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem $C_8$-$C_{30}$-Alkylrest terminiert ist. Dazu zählen z. B. Acrylat/Beheneth-25-methacrylat-Copolymere, die als Aculyn® (Rohm und Haas) kommerziell erhältlich sind.

In einer weiteren Ausführungsform liegt die erfindungsgemäße Zubereitung in Form eines Haarwachses vor, d.h. sie weist wachsartige Konsistenz auf und enthält mindestens eines der oben genannten Wachse in einer Menge von vorzugsweise 0,5 bis 30 Gew.% sowie gegebenenfalls weitere wasserunlösliche Stoffe. Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größer oder gleich 30°C und kleiner oder gleich 70°C ist, besonders bevorzugt im Bereich von 40 bis 55°C liegt. Geeignete Wachse und wasserunlösliche Stoffe sind insbesondere Emulgatoren mit einem HLB-Wert unterhalb von 7, Silikonöle, Silikonwachse, Wachse (2.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hydrophile Wachse wie z.B. hochmolekulare Polyethylenglykole mit einem Molekulargewicht von 800 bis 20.000, vorzugsweise von 2.000 bis 10.000 g/mol.

Wenn die erfindungsgemäße kosmetische, bevorzugt haarkosmetische Zubereitung in Form einer Haarlotion vorliegt, so liegt sie als im wesentlichen nicht-viskose oder gering viskose, fließfähige Lösung, Dispersion oder Emulsion mit einem Gehalt an mindestens 10 Gew.%, vorzugsweise 20 bis 95 Gew.% eines kosmetisch verträglichen Alkohols

vor. Als Alkohole können insbesondere die für kosmetische Zwecke üblicherweise verwendeten niederen Alkohole mit 1 bis 4 C-Atomen, z.B. Ethanol und Isopropanol verwendet werden.

Wenn die erfindungsgemäße haarkosmetische Zubereitung in Form einer Haarcreme vorliegt, so liegt sie vorzugsweise als Emulsion vor und enthält entweder zusätzlich viskositätsgebende Inhaltsstoffe in einer Menge von 0,1 bis 10 Gew.% oder die erforderliche Viskosität und cremige Konsistenz wird durch Micellbildung mit Hilfe von geeigneten Emulgatoren, Fettsäuren, Fettalkoholen oder Wachsen in üblicher Weise aufgebaut.

Die erfindungsgemäßen Copolymere können in kosmetischen Zubereitungen als Konditioniermittel eingesetzt werden.

Die erfindungsgemäßen Copolymere können bevorzugt in Shampooformulierungen als Festigungs- und/oder Konditioniermittel eingesetzt werden. Bevorzugte Shampooformulierungen enthalten

i) 0,05 bis 10 Gew.-% wenigstens eines erfindungsgemäßen Copolymers,
ii) 25 bis 94,95 Gew.-% Wasser,
iii) 5 bis 50 Gew.-% Tenside,
iv) 0 bis 5 Gew.-% eines weiteren Konditioniermittels,
v) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und

Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Polymeren A eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat®FC, Luviquat®HM, Luviquat®MS, Luviquat®Care, Luviquat®Ultracare), Copolymere aus N-Vinylpyrrolidon/Dimethylaminoethylmethacrylat, quaternisiert mit Diethylsulfat (Luviquat®PQ 11), Copolymere aus N-Vinylcaprolactam/N-Vinylpyrrolidon/N-Vinylimidazoliumsalzen (Luviquat®Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Siiikonverbindungen wie Amodimethicone (CTFA). Ferner können kationische Guarderivate wie Guarhydroxypropyltrimoniumchlorid (INCI) verwendet werden.

Messmethoden

Bestimmung des K-Wertes

Die K-Werte werden nach Fikentscher, Cellulosechemie, Bd. 13, S. 58 bis 64 (1932) bei 25°C in Ethanol- oder N-Methylpyrrolidon (NMP)-Lösung gemessen und stellen ein Maß für das Molgewicht dar. Die Ethanol- oder NMP-Lösungen der Polymere enthalten jeweils 1g des jeweiligen erfindungsgemäßen Copolymers in 100 ml Lösung.

Für den Fall, dass die Polymere in Form von wässrigen Dispersionen vorliegen, werden in Abhängigkeit vom Polymergehalt der Dispersion entsprechende Mengen der Dispersion mit Ethanol auf 100 ml aufgefüllt, so dass die Konzentration 1 g Polymer in 100 ml beträgt.

Die Messung des K-Wertes erfolgt in einer Micro-Ubbelohde-Kapillare Typ M Ic der Fa. Schott.

Bestimmumg der Tröpfchengrößenverteilung (TGV) mittels Malvern®-Streulichtanalyse Die Bestimmung der Tröpfchengrößenverteilung wurde mit Partikelgrößenmeßsystem zur Erfassung von Flüssigkeits-Aerosolen "Malvern®Master Sizer X" vorgenommen (Malvern Instruments Inc., Southborough MA, USA).

Messprinzip:

Das Messprinzip ist die Laserlicht-Beugung am Partikel, die sich außer zur Spray-Analyse (Aerosole, Pumpsprays) auch zur Größenbestimmung von Feststoffen, Suspensionen und Emulsionen im Größenbereich von 0,1 µm bis 2000µm eignet.

Ein Partikelkollektiv (=Tröpfchen) wird von einem Laser beleuchtet. An jedem Tröpfchen wird ein Teil des einfallenden Laserlichtes gestreut. Dieses Licht wird an einem Muttielement-Detektor empfangen und die dazugehörige Lichtenergieverteilung bestimmt. Aus diesen Daten wird über die Auswertesoftware die dazugehörige Partikelverteilung berechnet.

Durchführung:

Die Aerosole wurden in einem Abstand von 29,5cm zum Laserstrahl eingesprüht. Der Sprühkegel trat rechtwinklig zum Laserstrahl ein.

Die Aerosoldosen wurden vor jeder Messung an einer fest installierten Haltevorrichtung fixiert, somit wurde erreicht, dass alle zu prüfenden Aerosole im exakt gleichen Abstand vermessen wurden.

Vor der eigentlichen Partikelmessung wurde eine "Hintergrundmessung" durchgeführt. Dadurch wurden die Auswirkungen von Staub und anderen Verschmutzungen im Messbereich eliminiert.

Anschließend wurde das Aerosol in den Prüfraum eingesprüht. Das gesamte Partikelvolumen wurde über eine Prüfdauer von 2s erfasst und ausgewertet.

Auswertung:

Die Auswertung enthält eine tabellarische Darstellung über 32 Klassenbreiten von 0,5µm bis 2000µm und zusätzlich eine graphische Darstellung der Partikelgrößenverteilung.

Da es sich bei den Sprühversuchen um eine annähernd gleichmäßige Verteilung handelt, wird der mittlere Durchmesser "Mean Diameter" D(v,0.5) angegeben. Dieser Zahlenwert gibt an, dass 50% des gesamt gemessenen Partikelvolumens unterhalb dieses Wertes liegt.

Bei gut sprühbaren Aerosolsystemen im kosmetischen Bereich liegt dieser Wert, je nach Polymergehalt, Ventil- und Sprühkopfgeometrie, Lösungsmittelverhältnis und Treibgasmengen im Bereich von 30µm bis 80µm.

Bestimmung der Festigung (Biegesteifigkeit):

Die Festigung von polymeren Filmbildnern wurde außer durch subjektive Beurteilung (Handtest) auch physikalisch durch Messung der Biegesteifigkeit von dünnen Haarsträhnen (jeweils ca. 3 g und 24 cm Länge) gemessen. Dazu wurden die gewogenen, trockenen Haarsträhnen in die 3,0 Gew.-%ige Polymerlösung (Lösungsmittel: Ethanol/ Wasser 55:45 w/w) getaucht, wobei durch dreimaliges Eintauchen und Herausnehmen und anschließendes Ausdrücken zwischen Filterpapier eine gleichmäßige Benetzung der Haarsträhne und Verteilung der Polymerlösung sichergestellt wurde. Die überschüssige Filmbildnerlösung wurde dann zwischen Daumen und Zeigefinger abgestreift und die Haarsträhnen wurden die Strähnen von Hand so geformt, dass sie einen etwa runden Querschnitt erhielten. Bei 20°C und 65 % relativer Feuchte wurde über Nacht im Klimaraum getrocknet. Die Prüfungen wurden im Klimaraum bei ca. 20°C und ca. 65 % relativer Feuchte mittels eines Zug/Druck-Prüfgerätes durchgeführt. Die Haarsträhne wurde symmetrisch an den Enden auf zwei zylindrische Rollen der Probenaufnahme gelegt. Genau in der Mitte wurde die Strähne dann von oben mit einem abgerundetem Stempel ca.40 mm durchgebogen (Brechen des Polymerfilms). Die dafür erforderliche Kraft (Fmax) wurde mit einer Wägezelle (50 N) bestimmt. Dabei stellt ein Messwert das arithmetische Mittel aus den Einzelmessungen an 5 bis 10 gleich behandelten Hasrstähnen dar. Die so ermittelten Werte wurden zu denen eines handelsüblichen Vergleichspolymers (Amphomer®LV-71) ins Verhältnis gesetzt und in % angegeben.

Bestimmung der Auswaschbarkeit:

Eine analog zur Bestimmung der Festigung mit Polymer behandelte Haarsträhne wurde in einer ca. 37°C warmen Texapon®NSO - Lösung (6ml Texapon®NSO (28 %ig) in 11 warmes Wasser) ca. 15 Sekunden durch 5maliges Eintauchen und Ausdrücken gewaschen. Anschließend wurde die Haarsträhne klargespült und nochmals in der gleichen Art behandelt. Danach wurde die Haarsträhne auf Filterpapier gut ausgedrückt und über Nacht trocknen gelassen. Die trockene Haarsträhne wurde eingedreht und auf Rückstände untersucht.

Bestimmung der Curl Retention

Gründrezeptur: (Aerosol-Haarspray)

| | |
|---|---|
| 5 Gew.-% | Wirkstoff zu prüfendes Polymer (100% neutr. mit AMP) |
| 15 Gew.-% | Ethanol |
| 40 Gew.-% | Wasser |
| 40 Gew.-% | Dimethylether. |

Für die Bestimmung der Curl Retention wurden Haarsträhnen von ca. 2 g Gewicht und 15,5 cm Länge und aus mittelbraunem, kaukasischem Menschenhaar verwendet.

Behandlung der Haarsträhnen:

Die Haarsträhnen wurden zweimal mit einer wässrigen Texapon®NSO-Lösung gewaschen. Anschließend wurden die Haarsträhnen mit warmem Wasser ausgespült, bis keine Schaumbildung mehr erkennbar war und mit VE-Wasser nachgespült, gekämmt und auf Filterpapier zum Trocknen gelegt.

Zur Herstellung einer Wasserwelle werden die Haarsträhnen 15 Minuten zum Quellen in einer Lösung aus Ethanol und Wasser (1:1) eingelegt.

Die Haarsträhne wurde vor der Lockenpräparation sorgfältig gekämmt. Mit einem Gummiband wurde die Haarsträhne am Plexiglasstab befestigt. Anschließend wurde gekämmt und spiralförmig gewickelt. Mit einem Baumwolltuch und Gummiband wurde die Locke fest fixiert und über Nacht bei ca. 70°C getrocknet. Die abgekühlten Curl

Retention-Strähnen wurden vorsichtig geöffnet und vom Plexiglasstab abgestreift, ohne die Wasserwelle zu deformieren. Aus einer Entfernung von 15 cm wurden 1,8g des wie vorgenannt hergestellten Aerosol-Haarprays gleichmäßig auf die Locke ausgesprüht. Die Locke wurde dabei gleichmäßig gedreht. In horizontaler Lage wurden die Locken für 1 Stunde bei Raumtemperatur getrocknet. Nach der Trocknung wurden die Locken in einer Halterung befestigt. Mit Hilfe eines Maßstabs wurde zu Beginn die Länge $L_0$ der Locken abgelesen und die Längenausdehnung während der Feuchtklimalagerung verfolgt. Nach 5h Lagerung bei 25°C und 90% relativer Feuchte (r.F.) in der Klimakammer wurde die erreichte Länge $L_t$ der Locke erneut abgelesen und die Curl Retention nach folgender Gleichung berechnet:

$$\text{Curl Retention in \%} = \frac{L - L_t}{L - L_0} * 100$$

$L$ = Länge der Haare (15,5 cm)
$L_0$ = Länge der Haarlocke nach dem Trocknen
$L_t$ = Länge der Haarlocke nach Klimabehandlung

Als Curl Retention wurde der Mittelwert aus 5 Einzelmessungen angegeben.

Bestimmung der Klebrigkeit

Es wurde zunächst eine klare, 20 Gew.-%ige ethanolische oder ethanolisch/wässrige Lösung des zu charakterisierenden Polymers hergestellt. Um eine klare Lösung zu erhalten, war es teilweise notwendig, das Polymer zu neutralisieren. Aus der ethanolischen oder ethanolisch/wässrigen Lösung wurde dann mit einem Rakel (120 µm Spaltbreite) ein Film des Polymers auf einer rechteckigen Glasplatte mit einer Länge von ca. 20 cm und einer Breite von ca.

6,5 cm aufgebracht. Der darauf aufgebrachte Polymerfilm hatte jeweils eine Länge von ca. 16 bis 18 cm und eine Breite von ca. 5,5 cm.

Der Film wurde dann ca. 10 Stunden an der Luft getrocknet und anschließend im Klimaschrank bei 20°C und 80% r.F. weitere 12 Stunden gelagert.

Bei diesen Bedingungen wurde dann im Klimaschrank ein sich auf einem runden Gummistempel (Durchmesser 400 mm, Shore A-Härte 60 ± 5) befindendes Plastic-Carbon-Band (z.B. Pelikan®2060, 50 mm breit) mit einer Kraft von ca. 250 N für 10 Sekunden auf den Polymerfilm gepresst.

Die Menge des nach Entfernen des Stempels auf dem Polymerfilm haftend verbliebenen Schwarz-Pigmentes entspricht der Klebrigkeit des Filmes. Es erfolgte eine visuelle Beurteilung der Schwarzfärbung des Filmes. Die Beurteilungsskala reicht von 0 bis 5, wobei 0 "nicht klebrig" und 5 "sehr stark klebrig" bedeuten.

Bestimmung des Aussehens der Aerosol-Formulierung

Die Zubereitung aus 5 Gew.-% des jeweiligen Polymers neutralisiert mit AMP, 40 Gew.-% Dimethylether, 15 Gew.-% Ethanol und 40 Gew.-% Wasser wurden in einen transparenten Glas-Aerosolbehälter gefüllt. Anschließend wurden die Klarheit des resultierenden Flüssigkeits/Treibgasgemisches visuell beurteilt.

Beispiele

Die folgenden Beispiele erläutern die Erfindung, ohne sie darauf zu beschränken. Die Prozentangaben bedeuten Gew.-%, sofern nicht anderweitig bezeichnet.

Verwendete Abkürzungen:

| | |
|---|---|
| t-BA | tert.-Butylacrylat |
| t-BMA | tert.-Butyl(meth)acrylat |
| HEMA | 2-Hydroxyethylmethacrylat |
| HPMA | 2-Hydroxypropylmethacrylat |
| MAS | Methacrylsäure |
| AS | Acrylsäure |

Beispiel 1: tert.-Butylacrylat/Methacrylsäure 75/25 w/w (Vergleichsbeispiel)

In einem 2 l -Polymerisationsgefäß mit Rührer sowie Heiz- und Kühleinrichtungen wurden bei einer Temperatur von 20 bis 25 °C

| | |
|---|---|
| 250 | g entionisiertes Wasser |
| 0,6 g | einer 15 gew.-%igen wässrigen Lösung von Natriumlaurylsulfat in entionisiertem Wasser |
| 35g | von Zulauf II (siehe unten) |

vorgelegt und unter Rühren und Stickstoffatmosphäre auf 45°C aufgeheizt. Nach Erreichen der Temperatur wurde Zulauf I (siehe unten) innerhalb von 5 Minuten zugegeben.

Anschließend wurde auf 80°C aufgeheizt und unter Rühren und Beibehaltung der Reaktionstemperatur der restliche Zulauf II innerhalb von 2,5 Stunden mit gleichbleibenden Zulaufströmen zudosiert.

Nach Ende der Zuläufe wurde das Reaktionsgemisch für eine weitere Stunde bei 80°C gerührt und dann auf 60°abgekühlt.

Unter Beibehaltung der Temperatur von 60° wurde Zulauf III (siehe unten) zugegeben. Anschließend wurde auf 35°C abgekühlt und unter Beibehaltung der Reaktionstemperatur Zulauf IV (siehe unten) innerhalb von 60 Minuten zugegeben.

Zulauf I:

5 g einer 7 gew.-%ige wässrige Lösung von Natriumpersulfat in entionisiertem Wasser

Zulauf II ist eine wässrige Monomerenemulsion hergestellt aus:

| Einwaage [g] | | Gew.-% bezogen auf Gesamtmonomermenge |
|---|---|---|
| 120 | entionisiertem Wasser | |
| 5 | einer 15 gew.-%igen wässrigen Lösung von Natriumlaurylsulfat in entionisiertem Wasser | |
| 10 | nichtionischem Emulgator* | |
| 182 | tert.-Butylacrylat | 75 |
| 61 | Methacrylsäure | 25 |
| 1,8 | n-Dodecylmercaptan | |
| * als nichtionischer Emulgator kann beispielsweise TweenTM 80 eingesetzt werden. | | |

Herstellung Zulauf II

Zu dem vorgelegten, entionisierten Wasser gibt man unter Rühren die Gesamtmenge der 15 gew.-%igen wässrigen Lösung von Natriumlaurylsulfat und anschließend die Gesamtmenge des nichtionischen Emulgators. Zu der homogenen Lösung, die weiterhin gerührt wird, werden in der angegebenen Reihenfolge die entsprechenden Mengen tert.-Butylacrylat, Methacrylsäure und n-Dodecylmercaptan zugegeben.

Zulauf III:

2 g einer 30 gew%-ige Lösung von Wasserstoffperoxid in entionisiertem Wasser

Zulauf IV:

40 g einer 10 gew.%ige Lösung von Ammoniumhydrogencarbonat in entionisiertem Wasser

Die Polymere der folgenden Beispiele 2 bis 7 wurden analog zu Beispiel 1 synthetisiert, wobei Zulauf II für jedes Beispiel wie unten angegeben entsprechend gewählt wurde. Emulgatoren/Wasser/Ansatzgröße/Emulsionsherstellung/Regler analog Beispiel 1.

Beispiel 8: Herstellung von Polymer 8 (Lösungspolymerisation in Ethanol)

Unter Rühren bei 20°C wurden die folgenden Zuläufe hergestellt:

Zulauf 1:

| | |
|---|---|
| 123 g | t-BA |
| 55 g | t-BMA |
| 41g | HEMA |
| 15 g | MAS |
| 5 g | AS |
| 200 g | Ethanol |

Zulauf 2 :

| | |
|---|---|
| 7 g | Wako V 59 |
| 50 g | Ethanol |

Bei 20°C wurden eine Mischung aus 300g Ethanol, 15% der Gesamtmenge von Zulauf 1 sowie 15% der Gesamtmenge von Zulauf 2 hergestellt. Die Mischung wurde unter Normaldruck auf 78°C erhitzt. Nach Erreichen von 78°C wurden Zulauf 1 und Zulauf 2 gleichzeitig gestartet. Zulauf 1 wurde innerhalb von 3h sowie Zulauf 2 innerhalb von 4h mit gleichbleibendem Zulaufstrom zudosiert. Die Reaktionsmischung wurde während des gesamten Zulaufes bei 78 °C gehalten. Nach Ende des Zulaufes 2 wurde die Reaktionsmischung noch weitere 2h bei 78°C gehalten, anschließend wurde auf Raumtemperatur gekühlt.

Beispiel 9: Herstellung von Polymer 9 (Lösungspolymerisation in Ethanol/Wasser mit wasserlöslichen Startern)

Unter Rühren bei 20°C wurden die folgenden Zuläufe hergestellt:

| | |
|---|---|
| Zulauf 1: | |
| 90 g | MMA |
| 30 g | HEMA |
| 30 g | MAS |
| 200g | Ethanol |
| | |
| Zulauf 2 : | |
| 3 g | Natriumperoxodisulfat |
| 135 g | Ethanol |
| 102 g | Wasser |

Bei 20°C wurden eine Mischung aus 60g Ethanol, 45 g Wasser, 15% der Gesamtmenge von Zulauf 1 sowie 15% der Gesamtmenge von Zulauf 2 hergestellt. Die Mischung wurde unter Normaldruck auf 78°C erhitzt. Nach Erreichen von 78°C wurden Zulauf 1 und Zulauf 2 gleichzeitig gestartet. Zulauf 1 wurde innerhalb von 3h sowie Zulauf 2 innerhalb von 4h mit gleichbleibendem Zulaufstrom zudosiert. Die Reaktionsmischung wurde während des geamten Zulaufes bei 78 °C gehalten. Nach Ende des Zulaufes 2 wurde die Reaktionsmischung noch weitere 2h bei 78°C gehalten, anschließend wurde auf Raumtemperatur gekühlt.

Angabe der Monomerenzusammensetzung in Gewichtsprozent.

| Polymer aus Beispiel | t-BA | t-BMA | HEMA | HPMA | MAS | andere |
|---|---|---|---|---|---|---|
| 1 (Vergleichsbeispiel) | 75 | | | | 25 | |
| 2 | 65 | | 15 | | 20 | |
| 3 | 65 | | 10 | | 25 | |
| 4 | 50 | | 30 | | 20 | |
| 5 | 65 | | | 15 | 20 | |
| 6 | 50 | | | 30 | 20 | |
| 7 | 45 | 20 | 15 | | 20 | |
| 8 | 45 | 20 | 15 | | 15 | 5 AS |
| 9 | 60 | | 20 | | 20 | |

Anwendungstechnische Eigenschaften

| Polymer aus Beispiel | Klarheit im Aerosol*** | Biegesteifigkeit** [%] | TGV-Malvern [μm] | Curl-Retention [%] |
|---|---|---|---|---|
| 1* | klar | 73 | 73 | 47 |
| 2 | klar | 95 | 70 | 70 |

(fortgesetzt)

| Polymer aus Beispiel | Klarheit im Aerosol*** | Biegesteifigkeit** [%] | TGV-Malvern [μm] | Curl-Retention [%] |
|---|---|---|---|---|
| 3 | klar | 95 | 69 | 86 |
| * Vergleichsbeispiel<br>** Bestimmung der Festigung mittels Biegesteifigkeit-Methode (s. Messmethoden)<br>*** VOC55 Aerosol:<br>5% des jeweiligen Polymers, vollständig neutralisiert mit AMP,<br>40% DME,<br>15% Ethanol,<br>40% Wasser; | | | | |

Sprühvorrichtung:

Sprühkopf: Kosmos .020D Wirbel .018" 21-6443-20 (Fa. Precision Valve),
Ventil: DPV 33876 (Fa. Precision Valve)

Mit den Polymeren der Beispiele 2 und 3 werden bessere Festigungswirkung und Curl-Retention erzielt als mit dem Polymer des Beispiels 1 (Vergleichsbeispiel).

II. Anwendungstechnische Beispiele (AWB)

Falls nicht anders angegeben, werden alle eingesetzten Säuregruppen haltigen Polymere zu 100% neutralisiert mit AMP eingesetzt. "Wasser ad 100" bedeutet, dass zu der entspr. Formulierung die zum Erreichen einer Gesamtmenge von 100% notwendige Restmenge an Wasser zugegeben wird

**AWB 1:**

VOC 55-Aerosol-Haarspray

| | [%] |
|---|---|
| Polymer aus Beispiel Nr. 2 (fest) | 5,00 |
| Dimethylether | 40,00 |
| Ethanol | 15,00 |
| Wasser | ad 100 |
| weitere Zusätze: Silikon, Parfüm, Entschäumer, UV-Absorber... | |

Das Beispiel lässt sich jeweils mit den erfindungsgemäßen Polymeren 3-9 und Mischungen dieser Polymere wiederholen. Es wird jeweils ein VOC 55-Aerosol-Haarspray mit guten Eigenschaften erhalten.

**AWB 2:**

VOC 55-Aerosol-Haarspray

| | [%] |
|---|---|
| Polymer aus Beispiel Nr. 2 (fest) | 2,00 |
| Polymer aus Beispiel Nr. 3 (fest) | 2,00 |
| Dimethylether | 40,00 |
| Ethanol | 15,00 |

(fortgesetzt)

| | [%] |
|---|---|
| Wasser | ad 100 |
| weitere Zusätze: Silikon, Parfüm, Entschäumer, UV-Absorber ... | |

Das Beispiel lässt sich jeweils mit den erfindungsgemäßen Polymeren 4-9 und entspr. Mischungen der einzelnen Polymere wiederholen. Es wird jeweils ein VOC 55-Aerosol-Haarspray mit guten Eigenschaften erhaltene.

**AWB 3:**

VOC 55-Aerosol-Haarspray

| | [%] |
|---|---|
| Polymer aus Beispiel Nr. 2 (fest) | 5,00 |
| Dimethylether | 35,00 |
| Propan/Butan | 5,00 |
| Ethanol | 15,00 |
| Wasser | ad 100 |
| weitere Zusätze: Silikon, Parfüm, Entschäumer, UV-Absorber ... | |

Das Beispiel lässt sich jeweils mit den erfindungsgemäßen Polymeren 3-9 und Mischungen dieser Polymere wiederholen. Es wird jeweils ein VOC 55-Aerosol-Haarspray mit guten Eigenschaften erhalten.

**AWB 4:**

Aerosol-Haarspray mit Fluorcarbon-Treibmitteln

| | [%] |
|---|---|
| Polymer aus Beispiel Nr. 2 (fest) | 5,00 |
| Ethanol abs. | ad 100 |
| HFC 152A | 40,00 |
| weitere Zusätze: Silikon, Parfüm, Entschäumer, UV-Absorber ... | |

Das Beispiel lässt sich jeweils mit den erfindungsgemäßen Polymeren 3-9 und Mischungen dieser Polymere wiederholen. Es wird jeweils ein Aerosol-Haarspray mit guten Eigenschaften erhalten.

**AWB 5:**

Aerosol-Haarspray mit Fluorcarbon-Treibmitteln

| | [%] |
|---|---|
| Polymer aus Beispiel Nr. 2 (fest) | 5,00 |
| Dest. Wasser | ad 100 |
| HFC 152A | 10,00 |
| Dimethylether | 30,00 |
| Ethanol abs. | 30,00 |
| weitere Zusätze: Silikon, Parfüm, Entschäumer, UV-Absorber ... | |

Das Beispiel lässt sich jeweils mit den erfindungsgemäßen Polymeren 3-9 und Mischungen dieser Polymere wiederholen. Es wird jeweils ein Aerosol-Haarspray mit guten Eigenschaften erhalten.

**AWB 6:**

VOC 55-Aerosol-Haarspray

| | [%] |
|---|---|
| Polymer aus Beispiel Nr. 2 (fest) | 3,00 |
| Ultrahold® Strong (fest, Fa. BASF) | 1,00 |
| Dimethylether | 40,00 |
| Ethanol | 15,00 |
| + AMP | auf pH 8,3 |
| Wasser | ad 100 |
| weiterer Zusatz: Silikon, Parfüm, Entschäumer ... | |

Das Beispiel lässt sich jeweils mit den erfindungsgemäßen Polymeren 3-9 und Mischungen dieser Polymere wiederholen. Es wird jeweils ein VOC 55-Aerosol-Haarspray mit guten Eigenschaften erhalten.

**AWB 7:**

VOC 80-Aerosol-Haarspray

| | [%] |
|---|---|
| Polymer aus Beispiel Nr. 2 (fest) | 12,00 |
| Dimethylether | 40,00 |
| Ethanol | 40,00 |
| Wasser | ad 100 |
| weiterer Zusatz: Silikon, Parfüm, Entschäumer ... | |

Das Beispiel lässt sich jeweils mit den erfindungsgemäßen Polymeren 3-9 und Mischungen dieser Polymere wiederholen. Es wird jeweils ein VOC 80-Aerosol-Haarspray mit guten Eigenschaften erhalten.

**AWB 8:**

| Wässriges Handpumpenspray | [%] |
|---|---|
| Polymer aus Beispiel Nr. 2 (fest) | 4,00 |
| Luviset Clear *) (fest) | 1,00 |
| Wasser | ad 100 |
| weiterer Zusatz: wasserlösliches Silikon, Parfüm, Entschäumer ... | |

*) Luviset® Clear: Poly(Vinylpyrrolidon/Methacrylsäureamid/Vinylimidazol), Fa. BASF

Das Beispiel lässt sich jeweils mit den erfindungsgemäßen Polymeren 3-9 und Mischungen dieser Polymere wiederholen. Es wird jeweils ein wässriges Handpumpenspray mit guten Eigenschaften erhalten.

**AWB 9:**

| Wässrig/ethanolische Festiger-Lösung | [%] |
|---|---|
| Polymer aus Beispiel Nr. 2 (fest) | 7,0 |
| dest. Wasser | ad 100 |
| Ethanol | 52,00 |
| weiterer Zusatz: Silikon, Parfüm, Entschäumer ... | |

Das Beispiel lässt sich jeweils mit den erfindungsgemäßen Polymeren 3-9 und Mischungen dieser Polymere wiederholen. Es wird jeweils eine Festiger-Lösung mit guten Eigenschaften erhalten.

**AWB 10:**

| Ethanolische Festiger-Lösung | [%] |
|---|---|
| Polymer aus Beispiel Nr. 2 (fest) | 7,0 |
| Ethanol | ad 100 |
| weiterer Zusatz: Silikon, Parfüm, Entschäumer ... | |

Das Beispiel lässt sich jeweils mit den erfindungsgemäßen Polymeren 3-9 und Mischungen dieser Polymere wiederholen. Es wird jeweils eine ethanolische Festiger-Lösung mit guten Eigenschaften erhalten.

**AWB 11:**

| Haargel | [%] |
|---|---|
| Phase 1: | |
| Polymer aus Beispiel Nr. 2 (fest) | 6,00 |
| Wasser, dest. | ad 50 |
| weiterer Zusatz: Konservierungsmittel, lösliches ethoxiliertes Silikon, Parfüm ... | |
| | |
| Phase 2: | |
| Natrosol HR 250 (5 %ige Lösung) | 50,00 |
| Hydroxyethylcellulose (Fa. Hercules) | |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich jeweils mit den erfindungsgemäßen Polymeren 3-9 und Mischungen dieser Polymere wiederholen. Es wird jeweils ein Haarfestigergel mit guten Eigenschaften erhalten.

**AWB 12:**

| Schaumconditioner | [%] |
|---|---|
| Polymer aus Beispiel Nr. 2 (fest) | 0,50 |
| Cremophor® A 25 (Ceteareth 25/Fa. BASF) | 0,20 |
| Comperlan KD (Coamide DEA/Fa. Henkel) | 0,10 |

(fortgesetzt)

| Schaumconditioner | [%] |
|---|---|
| Propan/Butan | 10,00 |
| weiterer Zusatz: Parfüm, Konservierungsmittel .... | |
| Wasser | ad 100 |

Herstellung: Einwiegen und unter Rühren lösen. Abfüllen und Treibgas zusetzen.

Das Beispiel lässt sich jeweils mit den erfindungsgemäßen Polymeren 3-9 und Mischungen dieser Polymere wiederholen. Es wird jeweils ein Schaumconditioner mit guten Eigenschaften erhalten.

**AWB 13:**

| Conditionershampoo: | [%] |
|---|---|
| A) Texapon NSO 28 %ig (Natriumlaurethsulfat/Fa. Henkel) | 50,00 |
| Comperlan KS (Coamid DEA/Fa. Henkel) | 1,00 |
| Polymer aus Beispiel Nr. 2 (fest) | 3,00 |
| q. s. Parfümöl | |
| B) Wasser . | 44,5 |
| Natriumchlorid | 1,5 |
| q. s. Konservierungsmittel ... | |

Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich jeweils mit den erfindungsgemäßen Polymeren 3-9 und Mischungen dieser Polymere wiederholen. Es wird jeweils ein Conditionershampoo mit guten Eigenschaften erhalten.

**AWB 14:**

Standard O/W-Creme:

| Ölphase: | [%] | CTFA-Name |
|---|---|---|
| Crempophor A6 | 3,5 | Ceteareth-6 (and) Stearyl Alcohol |
| Crempophor A25 | 3,5 | Ceteareth-25 |
| Glycerinmonostearat s.e. | 2,5 | Glycerylstearat |
| Paraffinöl | 7,5 | Paraffin Oil |
| Cetylalkohol | 2,5 | Cetyl Alcohol |
| Luvitol EHO | 3,2 | Cetearyl Octanoate |
| Vitamin-E-Acetat | 1,0 | Tocopheryl Acetate |
| Nip-Nip | 0,1 | Methyl- and Propyl-4-hydroxybenzoate (7:3) |

Wasserphase:

| | |
|---|---|
| Polymer aus Beispiel Nr. 2 (fest) | 0,6 |

(fortgesetzt)

| | | |
|---|---|---|
| Polymer aus Beispiel Nr. 2 | | |
| Wasser | 77,0 | |
| 1,2-Propylenglykol | 1,5 | Propylenglykol |
| Germall II | 0,1 | Imidazolidinyl-Harnstoff |

Herstellung:

Die Öl- und Wasserphasen werden getrennt eingewogen und bei einer Temperatur von ca. 80 °C homogenisiert. Dann wird die Wasserphase langsam in die Ölphase eingerührt und unter Rühren langsam auf Raumtemperatur abgekühlt.

Das Beispiel lässt sich jeweils mit den erfindungsgemäßen. Polymeren 3-9 und Mischungen dieser Polymere wiederholen. Es wird jeweils eine Standard O/W-Creme mit guten Eigenschaften erhalten.

**AWB 15:**

Flüssiges Makeup

| | |
|---|---|
| A | |
| 1,70 | Glycerylstearat |
| 1,70 | Cetylalkohol |
| 1,70 | Ceteareth-6 |
| 1,70 | Ceteareth-25 |
| 5,20 | Capryl/Caprin-Triglycerid |
| 5,20 | Mineralöl |
| | |
| B | |
| q.s. | Konservierungsmittel |
| 4,30 | Propylenglykol |
| 2,50 | Polymer 2 (fest) |
| ad 100 | dest. Wasser |
| | |
| C | |
| q.s. | Parfumöl |
| | |
| D | |
| 2,00 | Eisenoxid |
| 12,00 | Titandioxid |

Herstellung:

Phase A und Phase B getrennt voneinander auf 80 °C erwärmen. Dann Phase B in Phase A mit einem Rührer mischen. Alles auf 40 °C abkühlen lassen und Phase C und Phase D zugeben. Wiederholt homogenisieren.

Das Beispiel lässt sich jeweils mit den erfindungsgemäßen Polymeren 3-9 und Mischungen dieser Polymere wiederholen. Es wird jeweils ein Make-Up mit guten Eigenschaften erhalten.

**Patentansprüche**

**1.** Copolymer, welches

a) zwischen 25 und 80 Gew.-% tert.-Butyl(meth)acrylat,

b) 2 bis 60 Gew.-% Hydroxyalkyl(meth)acrylat,
c) 10 bis 40 Gew.-% einer anionischen oder anionogenen, radikalisch polymerisierbaren Verbindung, die Methacrylsäure ist oder umfasst,
d) 0 bis 30 Gew.-% wenigstens einer weiteren radikalisch polymerisierbaren Verbindung

einpolymerisiert enthält, wobei sich die Mengen der Komponenten a) bis d) zu 100 Gew.-% addieren.

**2.** Copolymer nach Anspruch 1, welches

a) von mehr als 30 bis 75 Gew.-% Komponente a),
b) 5 bis 40 Gew.-% Komponente b),
c) 12 bis 35 Gew.-% Komponente c),
d) 0 bis 30 Gew.-% wenigstens einer weiteren radikalisch polymerisierbaren Verbindung

einpolymerisiert enthält, wobei sich die Mengen der Komponenten a) bis d) zu 100 Gew.-% addieren.

**3.** Copolymer nach einem der Ansprüche 1 oder 2, welches

a) 40 bis 70 Gew.-% Komponente a),
b) 10 bis 35 Gew.-% Komponente b),
c) 15 bis 30 Gew.-% Komponente c),
d) 0 bis 30 Gew.-% wenigstens einer weiteren radikalisch polymerisierbaren Verbindung

einpolymerisiert enthält, wobei sich die Mengen der Komponenten a) bis d) zu 100 Gew.-% addieren.

**4.** Copolymer nach einem der Ansprüche 1 bis 3, wobei

a) tert.-Butylacrylat ist oder umfasst,
b) Hydroxyethylmethacrylat ist oder umfasst,
c) Methacrylsäure ist oder umfasst.

**5.** Verwendung eines Copolymers gemäß einem der Ansprüche 1 bis 4 in kosmetischen Zubereitungen.

**6.** Kosmetische Zubereitung enthaltend ein Copolymer gemäß einem der Ansprüche 1 bis 4.

**7.** Kosmetische Zubereitung nach Anspruch 6, wobei der Anteil an flüchtigen organischen Komponenten höchstens 55 Gew.-%, bezogen auf die kosmetische Zubereitung, beträgt.

**8.** Kosmetische Zubereitung nach einem der Ansprüche 6 oder 7, wobei die Zubereitung weiterhin wenigstens einen kosmetisch akzeptablen Träger B aufweist, der ausgewählt ist unter

i) wassermischbaren organischen Lösungsmitteln, bevorzugt $C_2$-$C_4$-Alkanolen, besonders bevorzugt Ethanol,
ii) Ölen, Fetten, Wachsen,
iii) von ii) verschiedenen Estern von $C_6$-$C_{30}$-Monocarbonsäuren mit ein-, zwei- oder dreiwertigen Alkoholen,
iv) gesättigten acyclischen und cyclischen Kohlenwasserstoffen,
v) Fettsäuren,
vi) Fettalkoholen,
vii) Treibmitteln (Treibgasen) und
viii) Mischungen davon.

**9.** Kosmetische Zubereitung nach einem der Ansprüche 6 bis 8 in Form eines Sprühproduktes, wobei die Zubereitung entweder in Kombination mit einer mechanischen Pumpsprühvorrichtung oder in Kombination mit mindestens einem Treibmittel ausgewählt aus der Gruppe bestehend aus Propan, Butan, Dimethylether, fluorierten Kohlenwasserstoffen und deren Mischungen vorliegt.

## Claims

1. A copolymer, which comprises, in copolymerized form,

    a) between 25 and 80% by weight of tert-butyl (meth)acrylate,
    b) 2 to 60% by weight of hydroxyalkyl (meth)acrylate,
    c) 10 to 40% by weight of an anionic or anionogenic, free-radically polymerizable compound which is or comprises methacrylic acid,
    d) 0 to 30% by weight of at least one further free-radically polymerizable compound,

    where the amounts of the components a) to d) add up to 100% by weight.

2. The copolymer according to claim 1, which comprises, in copolymerized form,

    a) from more than 30 to 75% by weight of component a),
    b) 5 to 40% by weight of component b),
    c) 12 to 35% by weight of component c),
    d) 0 to 30% by weight of at least one further free-radically polymerizable compound,

    where the amounts of the components a) to d) add up to 100% by weight.

3. The copolymer according to one of claims 1 or 2, which comprises, in copolymerized form,

    a) 40 to 70% by weight of component a),
    b) 10 to 35% by weight of component b),
    c) 15 to 30% by weight of component c),
    d) 0 to 30% by weight of at least one further free-radically polymerizable compound,

    where the amounts of the components a) to d) add up to 100% by weight.

4. The copolymer according to one of claims 1 to 3, where

    a) is or comprises tert-butyl acrylate,
    b) is or comprises hydroxyethyl methacrylate,
    c) is or comprises methacrylic acid.

5. The use of a copolymer according to one of claims 1 to 4 in cosmetic preparations.

6. A cosmetic preparation comprising a copolymer according to one of claims 1 to 4.

7. The cosmetic preparation according to claim 6, where the fraction of volatile organic components is at most 55% by weight, based on the cosmetic preparation.

8. The cosmetic preparation according to one of claims 6 or 7, where the preparation also has at least one cosmetically acceptable carrier B, which is chosen from

    i) water-miscible organic solvents, preferably $C_2$-$C_4$-alkanols, particularly preferably ethanol,
    ii) oils, fats, waxes,
    iii) esters of $C_6$-$C_{30}$-monocarboxylic acids being different from ii) with mono, di- or trihydric alcohols,
    iv) saturated acyclic and cyclic hydrocarbons,
    v) fatty acids,
    vi) fatty alcohols,
    vii) propellants (propellant gases) and
    viii) mixtures thereof.

9. The cosmetic preparation according to one of claims 6 to 8 in the form of a spray product, where the preparation is present either in combination with a mechanical pump spray device or in combination with at least one propellant chosen from the group consisting of propane, butane, dimethyl ether, fluorinated hydrocarbons and mixtures thereof.

## Revendications

1. Copolymère qui contient, incorporés par polymérisation

   a) entre 25 et 80 % en poids de (méth)acrylate de tert-butyle,
   b) 2 à 60 % en poids de (méth)acrylate d'hydroxyalkyle,
   c) 10 à 40 % en poids d'un composé anionique ou anionogène, polymérisable par voie radicalaire, qui est ou comprend l'acide méthacrylique,
   d) 0 à 30 % en poids d'au moins un autre composé polymérisable par voie radicalaire,

   la somme des quantités des composants a) à d) étant égale à 100 % en poids.

2. Copolymère selon la revendication 1, qui contient, incorporés par polymérisation

   a) de plus de 30 à 75 % en poids de composant a),
   b) 5 à 40 % en poids de composant b),
   c) 12 à 35 % en poids de composant c),
   d) 0 à 30 % en poids d'au moins un autre composé polymérisable par voie radicalaire,

   la somme des quantités des composants a) à d) étant égale à 100 % en poids.

3. Copolymère selon la revendication 1 ou 2, qui contient, incorporés par polymérisation

   a) 40 à 70 % en poids de composant a),
   b) 10 à 35 % en poids de composant b),
   c) 15 à 30 % en poids de composant c),
   d) 0 à 30 % en poids d'au moins un autre composé polymérisable par voie radicalaire,

   la somme des quantités des composants a) à d) étant égale à 100 % en poids.

4. Copolymère selon l'une quelconque des revendications 1 à 3, dans lequel

   a) est ou comprend l'acrylate de tert-butyle,
   b) est ou comprend le méthacrylate d'hydroxyéthyle,
   c) est ou comprend l'acide méthacrylique.

5. Utilisation d'un copolymère selon l'une quelconque des revendications 1 à 4 dans des préparations cosmétiques.

6. Préparation cosmétique contenant un copolymère selon l'une quelconque des revendications 1 à 4.

7. Préparation cosmétique selon la revendication 6, dans laquelle la proportion de composants organiques volatils est d'au maximum 55 % en poids, par rapport à la préparation cosmétique.

8. Préparation cosmétique selon la revendication 6 ou 7, la préparation comportant en outre au moins un véhicule B cosmétiquement acceptable qui est choisi parmi

   i) des solvants organiques miscibles à l'eau, de préférence des alcanols en $C_2$-$C_4$, de façon particulièrement préférée l'éthanol,
   ii) des huiles, des graisses, des cires,
   iii) des esters d'acides monocarboxyliques en $C_6$-$C_{30}$, différents de ii), avec des alcools mono-, di- ou trihydriques,
   iv) des hydrocarbures saturés acycliques ou cycliques,
   v) des acides gras,
   vi) des alcools gras,
   vii) des propulseurs (gaz propulseurs) et
   viii) des mélanges de ceux-ci.

9. Préparation cosmétique selon l'une quelconque des revendications 6 à 8, sous forme d'un produit à pulvériser, la préparation se trouvant soit en association avec un pulvérisateur mécanique à pompe, soit en association avec au

moins un propulseur choisi dans le groupe constitué par le propane, le butane, l'éther diméthylique des hydrocarbures fluorés et des mélanges de ceux-ci.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1928368
- EP 379082 A
- EP 638306 A
- EP 705595 A
- EP 605686 A
- EP 985401 A
- EP 985405 A
- US 6214328 B
- EP 1321130 A
- US 3577518 A
- US 4196190 A
- US 5589157 A
- WO 0238638 A
- DE 4003422 A
- US 4269749 A
- EP 3957 B
- EP 28348 B
- EP 563726 B
- EP 764699 A
- EP 76180 A
- DE 3718520 A
- DE 3834734 A
- DE 4232194 A
- DE 19529599 A
- DE 19741187 A
- DE 19839199 A
- DE 19840586 A
- WO 9533775 A
- US 4529753 A
- EP 805169 A
- WO 9732917 A
- EP 1035144 A
- DE 4333238 A
- DE 3929973
- DE 2150557
- DE 2817369
- DE 3708451
- DE 3314742 A
- EP 1084696 A
- DE PS1165574 C
- DE PS2024051 C
- EP 934956 A


### In der Beschreibung aufgeführte Nicht-Patentliteratur


- Encyclopedia of Polymer Science and Engineering. John Wiley & Sons, Inc, 1987, vol. 8, 659-677
- **D.C. BLACKLEY.** Emulsion Polymerisation. Applied Science Publishers, Ltd, 1975, 155-465
- **D.C. BLACKLEY.** Polymer Latices. Chapman & Hall, 1997, vol. 1, 33, 415
- **H. WARSON.** The Applications of Synthetic Resin Emulsions. Ernest Benn, Ltd, 1972, 49-244
- **D. DIEDERICH.** Chemie. Verlag Chemie, 1990, vol. 24, 135-142
- **J. PIIRMA.** Emulsion Polymerisation. Academic Press, 1982, 1-287
- **F. HÖLSCHER.** Dispersionen synthetischer Hochpolymerer. Springer-Verlag, 1969, 1-160
- Methoden der organischen Chemie. **HOUBEN-WEYL.** Makromolekulare Stoffe. Georg-Thieme-Verlag, 1961, vol. XIV/1, 411-420
- Methoden der organischen Chemie. **HOUBEN-WEYL.** Makromolekulare Stoffe. Georg-Thieme-Verlag, 1961, vol. XIV/1, 192-208
- *Bestimmung nach Fikentscher, Cellulosechemie,* 1932, vol. 13, 58-64
- **KARL-HEINZ SCHRADER.** Grundlagen und Rezepturen der Kosmetika. Verlag Hüthig, 319-355
- **W. UMBACH.** Kosmetik und Hygiene von Kopf bis Fuß. Wiley-VCH, 2004, 235-236
- *Cellulosechemie,* 1932, vol. 13, 58-64